# EUROPEAN PATENT APPLICATION

(11) **EP 1 820 806 A1**
(43) Date of publication of application: **22.08.2007**
(21) Application number: 06075355.5
(22) Date of filing: 16.02.2006
(51) Int. Cl.: C07K 16/18, A61K 39/395, C07K 1/22, G01N 33/68, A61P 25/28

(54) **Affinity regions**

(71) Applicant: Crossbeta Biosciences B.V., 3584 CX Utrecht (NL)
(72) Inventor: Gebbink, Martijn Frans Ben Gerard, 3755 HL Eemnes (NL); Bouma, Barend, 3994 CE Houten (NL)
(74) Representative: Winckels, Johannes Hubertus F.

(57) **Abstract**

The present invention provides a method for selecting from a collection of IgIV molecules, at least one IgIV molecule comprising an affinity region that is capable of interacting with an epitope of a cross-β structure and/or with an epitope of a protein comprising a cross-β structure, said method comprising contacting a collection of IgIV molecules with a cross-β structure and/or a protein comprising a cross-β structure and collecting at least one IgIV molecule comprising an affinity region interacting with said epitope.

### Annex to the application documents - subsequently filed sequences listing

SEQUENCE LISTING

<110>  Crossbeta Biosciences B.V.

<120>  Affinity regions

<130>  P75584EP00

<140>  EP 06075355.5
<141>  2006-02-12

<160>  7

<170>  PatentIn version 3.3

<210>  1
<211>  13
<212>  PRT
<213>  Artificial Sequence

<220>
<223>  Synthetic human fibrin peptide

<400>  1

Lys Arg Leu Glu Val Asp Ile Asp Ile Gly Ile Arg Ser
1               5                   10


<210>  2
<211>  40
<212>  PRT
<213>  Artificial Sequence

<220>
<223>  Synthetic human amyloid-beta peptide

<400>  2

Asp Ala Glu Phe Arg His Asp Ser Gly Tyr Glu Val His His Gln Lys
1               5                   10                  15


Leu Val Phe Phe Ala Glu Asp Val Gly Ser Asn Lys Gly Ala Ile Ile
            20                  25                  30


Gly Leu Met Val Gly Gly Val Val
        35                  40


<210>  3
<211>  40
<212>  PRT
<213>  Artificial Sequence

<220>
<223>  Synthetic human A beta E22 of Dutch type

<400>  3

Asp Ala Glu Phe Arg His Asp Ser Gly Tyr Glu Val His His Gln Lys
1               5                   10                  15

Leu Val Phe Phe Ala Gln Asp Val Gly Ser Asn Lys Gly Ala Ile Ile
            20                  25                  30

Gly Leu Met Val Gly Gly Val Val
        35                  40

<210>  4
<211>  10
<212>  PRT
<213>  Artificial Sequence

<220>
<223>  Synthetic human fibrin fragment FP10

<400>  4

Lys Arg Leu Glu Val Asp Ile Asp Ile Lys
1               5                   10

<210>  5
<211>  7
<212>  PRT
<213>  Artificial Sequence

<220>
<223>  Peptide fragment NH2-GNNQQNY-COOH of yeast prion protein

<400>  5

Gly Asn Asn Gln Gln Asn Tyr
1               5

<210>  6
<211>  6
<212>  PRT
<213>  Artificial Sequence

<220>
<223>  Human fibrin peptide NH2-IDIKIR-COOH

<400>  6

Ile Asp Ile Lys Ile Arg
1               5

<210>  7
<211>  6
<212>  PRT
<213>  Artificial Sequence

<220>
<223>  Synthetic thrombin receptor activating peptide TRAP

<400> 7

Ser Phe Leu Leu Arg Asn
1               5

## Description

The invention relates to the fields of biochemistry, molecular biology, structural biology and medicine.

Immune Globulin Intravenous (IgIV) are immunoglobulins of apparently healthy animals or humans which immunoglobulins are collected from serum or blood. IgIV are prescribed to animals and humans that have a lack of antibodies. The cause of said lack of antibodies may be an illness affecting the immune system, such as for example AIDS, or an inborn error causing complete or partial a-gammaglobulinaemia or hypogammaglobulinaemia such as for example a primary immunodeficiency syndrome or severe combined immunodeficiency syndrome (SCIDS). Immunoglobulins collected from human serum or blood are commercially available under many different names such as for example "intravenous human immunoglobulins" (IgIV, or IVIg). Since IgIV were first introduced in 1981 for immunoglobulin substitution therapy of above-described immunodeficient humans, they have also been applied off label to patients suffering of a wide variety of diseases and in a number of cases, experimental treatment with IgIV turned out to be successful.

Because the mechanism of action has not been elucidated up to now, many of the off label treatments with IgIV were trial and error experiments, and the outcome was rather unpredictable.

The treatment with IgIV is not without risks. Since 1981, the Food and Drug Administration (FDA) has received over 114 worldwide (approximately 83 U.S.) adverse event reports of renal dysfunction and/or acute renal failure associated with the administration of these products. Although acute renal failure was successfully managed in the majority of cases, deaths were reported in 17 patients worldwide. Many of the patients who died had serious underlying conditions. For further reported side effects related to administering of IgIV in patients see Table 1.

In conclusion, the treatment of humans with IgIV is not clearly limited to a specific disease condition, a clear understanding of the mode of action is lacking and the outcome of an IgIV treatment for a new disease entity is unpredictable. The administration of large amounts of IgIV involves the risk of adverse side effects. Therefore, there is a need for improvement of current IgIV treatment.

It is an object of the present invention to provide means and methods for improving IgIV therapy. It is a further object to provide a selection and/or purification of a group of immunoglobulins from an IgIV pool, and an alternative mode of preparation of immunoglobulins or a functional equivalent thereof in order to obtain an immunoglobulin or equivalent thereof, suitable for treatment of a disease or a group of diseases, with at least one improved property as compared to IgIV, such as for instance a reduced risk of adverse side effects and/or an improved therapeutic action. Up to now, a person skilled in the art would not know how to start and which selection to make.

The present invention provides the insight that a selection of IgIV that is enriched in IgIV molecules capable of interacting with an epitope of a cross-β structure and/or with an epitope of a protein comprising a cross-β structure has improved properties as compared to currently used IgIV. Said selection is preferred over currently used IgIV, amongst other things because adverse side effects are at least in part prevented and/or therapeutic action is improved.

A cross-β structure is a secondary structural element in peptides and proteins. A cross-β structure (also referred to as a "cross-β", a "cross beta" or a "crossbeta" structure) is defined as a part of a protein or peptide, or a part of an assembly of peptides and/or proteins, which comprises an ordered group of β-strands, typically a group of β-strands arranged in a β-sheet, in particular a group of stacked β-sheets, also referred to as "amyloid". A typical form of stacked β-sheets is in a fibril-like structure in which the β-sheets are stacked in either the direction of the axis of the fibril or perpendicular to the direction of the axis of the fibril. The direction of the stacking of the β-sheets in cross-β structures is perpendicular to the long fiber axis. A cross-β structure conformation is a signal that triggers a cascade of events that induces clearance and breakdown of the obsolete protein or peptide. When clearance is inadequate, unwanted proteins and/or peptides aggregate and form toxic structures ranging from soluble oligomers up to precipitating fibrils and amorphous plaques. Such cross-β structure conformation comprising aggregates underlie various diseases, such as for instance, Huntington's disease, amyloidosis type disease, atherosclerosis, diabetes, bleeding, thrombosis, cancer, sepsis and other inflammatory diseases, rheumatoid arthritis, transmissible spongiform encephalopathies such as Creutzfeldt-Jakob disease, Multiple Sclerosis, auto-immune diseases, diseases associated with loss of memory such as Alzheimer's disease, Parkinson's disease and other neuronal diseases (epilepsy), encephalopathy and systemic amyloidoses.

A cross-β structure is for instance formed during unfolding and refolding of proteins and peptides. Unfolding of peptides and proteins occur regularly within an organism. For instance, peptides and proteins often unfold and refold spontaneously at the end of their life cycle. Moreover, unfolding and/or refolding is induced by environmental factors such as for instance pH, glycation, oxidative stress, heat, irradiation, mechanical stress, proteolysis and so on. The terms unfolding, refolding and misfolding relate to the three-dimensional structure of a protein or peptide. Unfolding means that a protein or peptide loses at least part of its three-dimensional structure. The term refolding relates to the coiling back into some kind of three-dimensional structure. By refolding, a protein or peptide can regain its native configuration, or an incorrect refolding can occur. The term "incorrect refolding" refers to a situation when a three-dimensional structure other than a native configuration is formed. Incorrect refolding is also called misfolding. Unfolding and refolding of proteins and peptides involves the risk of cross-β structure formation. Formation of cross-β structures sometimes also occurs directly after protein synthesis, without a correctly folded protein intermediate.

The term peptide is intended to include oligopeptides as well as polypeptides, and the term protein includes proteinaceous molecules with and without post-translational modifications such as glycosylation and glycation. It also includes lipoproteins and complexes comprising a proteinaceous part, such as protein-nucleic acid complexes (RNA and/or DNA), membrane-protein complexes, etc. As used herein, the term "protein" also encompasses proteinaceous molecules, peptides, oligopeptides and polypeptides.

According to the present invention, a selection of IgIV that is enriched in IgIV molecules capable of interacting with an epitope of a cross-β structure and/or with an epitope of a protein comprising a cross-β structure has at least one improved property as compared to currently used IgIV. The invention therefore provides a method for selecting from a collection of IgIV molecules at least one IgIV molecule capable of interacting with an epitope of a cross-β structure and/or with an epitope of a protein comprising a cross-β structure. This is preferably performed by contacting a collection of IgIV molecules with a cross-β structure and/or with a protein comprising a cross-β structure and collecting at least one IgIV molecule comprising an affinity region interacting with said epitope. Provided is therefore a method for selecting from a collection of IgIV molecules, at least one IgIV molecule comprising an affinity region that is capable of interacting with an epitope of a cross-β structure and/or with an epitope of a protein comprising a cross-β structure, said method comprising contacting a collection of IgIV molecules with a cross-β structure and/or a protein comprising a cross-β structure and collecting at least one IgIV molecule comprising an affinity region interacting with said epitope.

An affinity region influences the affinity with which a protein or peptide binds to an epitope and is herein defined as at least part of an antibody that is capable of specifically binding to an epitope. Said affinity region for instance comprises at least part of an immunoglobulin (such as in IgIV), at least part of a monoclonal antibody and/or at least part of a humanized antibody. Said affinity region preferably comprises at least part of a heavy chain and/or at least part of a light chain of an antibody. In one embodiment said affinity region comprises a double F(ab')₂ or single form Fab fragment.

Generally, affinity regions occur on the surface of cells such as T-cells or B-cells or other immune cells, in which case they are often part of a cellular receptor. Affinity regions also occur in synthetic form in phage display libraries.

One embodiment of the invention comprises contacting a collection of immunoglobulins of an IgIV solution with a collection of cross-β structures, preferably with a given selected cross-β structure, and/or with a protein comprising a cross-β structure, preferably a given selected protein comprising a cross-β structure. An epitope recognized by an affinity region is in one embodiment located on the cross-β structure itself. Therefore, one embodiment of the invention provides a method according to the invention for selecting from a collection of IgIV molecules, at least one IgIV molecule comprising an affinity region that is capable of interacting with an epitope of a cross-β structure and/or with an epitope of a protein comprising a cross-β structure, wherein said epitope is at least part of a cross-β structure of a protein. In another embodiment, said epitope is exposed on said protein comprising a cross-β structure. Said epitope is not necessarily located on said cross-β structure. Generally a cross-β structure induces a different folding of a protein, which often results in the induction and/or unveiling of hitherto unknown epitopes, or the change or deletion of known epitopes of said protein. Therefore, it is also possible that a protein in which a cross-β structure is formed during misfolding, displays an epitope that is related to the presence of a cross-β structure. In one embodiment an IgIV molecule capable of specifically binding such induced and/or unveiled epitope is selected.

As described before, cross-β structures and/or (misfolded) proteins comprising a cross-β structure are an underlying cause of disease symptoms of many diseases. Said disease symptoms, related to the presence of cross-β structures, are at least partly diminished by the administration of a collection of IgIV molecules according to the present invention. A collection of IgIV molecules according to the present invention is particularly suitable for removing proteins and/or peptides comprising a cross-β structure, preferably related to and/or associated with a disease, from a sample such as for instance a body fluid or tissue sample, thereby decreasing the amount of (circulating) proteins and/or peptides comprising a cross-β structure. As used herein, the term "removing a protein and/or peptide comprising a cross-β structure" comprises separating said protein and/or peptide from a sample, as well as binding, covering, shielding and/or neutralizing a cross-β structure and/or any other part of a protein or peptide comprising a cross-β structure, thereby at least in part preventing interaction of said cross-β structure and/or protein or peptide comprising a cross-β structure with other binding molecules. This way, adverse effects related to the presence of a cross-β structure and/or to the presence of a protein or peptide comprising a cross-β structure, such as for instance infections and/or inflammation in AIDS, and/or disease symptoms of such painful and devastating diseases like for example rheumatoid arthritis and multiple sclerosis, are at least in part decreased. The same principle is also applicable to inflammatory conditions in which proteins are altered by the presence of a cross-β structure (be it a cross-β structure generated by the body or generated and/or induced by a pathogen).

Because of the low concentration and the wide variety of antibodies reacting with a cross-β structure and/or with a protein comprising a cross-β structure, relatively large amounts of IgIV would have to be administered to achieve a positive result, which increases the risk of adverse side effects. Furthermore, in current IgIV treatments, the body is unnecessarily stressed by the administration of a lot of proteins that have no function for the disease they are administered for. It is therefore a major advantage that a selection is now made with a method according to the invention from the pool of IgIV for IgIV molecules capable of specifically binding a cross-β structure and/or a protein comprising a cross-β structure. IgIV preparations according to the invention comprising enriched fractions of immunoglobulins capable of specifically binding a cross-β structure and/or a protein comprising a cross-β structure are particularly suitable for administering to a non-human animal or human at risk of suffering or already suffering from a cross-β structure-related disease. Because now an enriched selection of IgIV is administered, comprising IgIV molecules capable of specifically reacting with a cross-β structure and/or with a protein comprising a cross-β structure, it has become possible to use a total concentration of IgIV molecules which is lower than in current IgIV treatments and still have the same or even better therapeutic effect than with currently used IgIV.

An IgIV molecule comprising an affinity region that is capable of interacting with an epitope of a cross-β structure and/or with an epitope of a protein comprising a cross-β structure is selected from a collection of IgIV molecules in various ways. For instance, said IgIV molecule is selected by contacting a pool of IgIV molecules with a cross-β structure and/or with a protein comprising a cross-β structure. Subsequently, bound IgIV molecules are collected. In one embodiment said cross-β structure and/or protein comprising a cross-β structure is related to a disease. For instance, myelin, myelin basic protein and/or myelin oligodendrocyte glycoprotein is preferably used in order to select IgIV molecules for use in at least in part treating and/or preventing multiple sclerosis. Likewise, collagen and/or rheuma factor is preferably used in order to select IgIV molecules for use in at least in part treating and/or preventing rheumatoid arthritis. Various alternative methods for selecting an IgIV molecule capable of interacting with an epitope of a cross-β structure and/or with an epitope of a protein comprising a cross-β structure are available in the art, which are suitable for use in a method according to the invention.

In one embodiment selected IgIV molecules are tested for their reactivity with a given protein and/or peptide of interest in a body sample of a human or animal suffering from said disease. The capability of a selected IgIV collection according to the invention of binding a specific protein of interest from a body sample is for example measured with a blood platelet aggregation test, an opsonophagocytosis test, and/or a complement activation or inhibition test.

One further embodiment provides a selection method according to the invention wherein an epitope, being a cross-β structure or an epitope of a protein comprising a cross-β structure, is attached to a support such as for example spheres or particles or beads or sheets or strands of latex or agarose or Sepharose or glass or plastic or metal or any other suitable substance or compound or material or molecule to enhance the efficiency of the selection, like for instance magnetic beads. Therefore the invention provides a method as described herein wherein said epitope is bound to a solid support.

The invention furthermore provides a collection of IgIV molecules, enriched in IgIV molecules comprising an affinity region that is capable of specifically interacting with an epitope of a cross-β structure and/or with an epitope of a protein comprising a cross-β structure. As explained above, said collection of IgIV molecules has at least one improved property as compared to currently used IgIV. A collection of IgIV molecules according to the invention is preferably selected from currently used IgIV with a selection method according to the invention. With a method of the invention, a skilled person is able to select from a large collection of IgIV, a smaller selection of IgIV molecules which is enriched in IgIV molecules comprising affinity regions capable of specifically binding an epitope on a cross-β structure and/or an epitope on a protein comprising a cross-β structure. Therefore, one embodiment provides a collection of IgIV molecules, enriched in IgIV molecules comprising an affinity region capable of interacting with an epitope of a cross-β structure and/or with an epitope of a protein comprising a cross-β structure, selected by a method according to the present invention. An enriched collection of IgIV molecules according to the invention is suitable for administering to a patient in need of such medicament in a smaller amount than currently used IgIV preparations, because of the relative increase of affinity regions in said enriched collection capable of interacting with an epitope on a cross-β structure and/or with an epitope on a protein comprising a cross-β structure.

The invention further provides a composition comprising at least 5 isolated, synthetic and/or recombinant molecules comprising an affinity region that is capable of interacting with an epitope of a cross-β structure and/or with an epitope of a protein comprising a cross-β structure. Preferably, said composition comprises at least 8, more preferably at least 10 of the above mentioned isolated, synthetic and/or recombinant molecules. In one preferred embodiment synthetic and/or recombinant molecules are used. An advantage of a composition of synthetic and/or recombinant molecules is the fact that the need for IgIV is obviated. This is, amongst other things, advantageous because the supplies and availability of IgIV are not sufficient, and because there are certain risks involved in administration of biological products derived from human blood (such as for instance the risk of infection with prion disease and with pathogens such as hepatitis virus or HIV). Now that the present invention has provided an enriched selection of IgIV molecules according to the invention, it has become possible to generate synthetic and/or recombinant molecules with at least one similar property as said enriched selection of IgIV molecules in kind, not necessarily in amount. Once an enriched selection of certain immunoglobulins of IgIV has been made, a skilled person is able to determine by methods known in the art (such as for example, but not limited to, the Maldi-Toff method) the amino acid sequence of said immunoglobulins, or at least of an affinity region of said immunoglobulins. Said amino acid sequence is then preferably used to select or produce synthetic or partially synthetic molecules that have the same binding characteristic in kind, not necessarily in amount, as at least one affinity region of a selected IgIV molecule according to the invention. A non-limiting example of a synthetic or partially synthetic molecule is a product obtained by recombinant or chemical synthesis of peptides, proteins or other molecules. It is even possible to screen a phage display library with cross-β structures or proteins comprising a cross-β structure, or epitopes of said proteins, to select binding molecules having an affinity region reacting with said cross-β structure and/or protein comprising a cross-β structure. Therefore, one embodiment provides a method for producing a composition according to the invention, comprising defining the amino acid sequence of an affinity region of at least one IgIV molecule capable of interacting with an epitope of a cross-β structure and/or with an epitope of a protein comprising a cross-β structure, and producing synthetic and/or recombinant molecules comprising said amino acid sequence. In another embodiment, the invention also provides a synthetic or recombinant molecule comprising an affinity region that is capable of interacting with an epitope of a cross-β structure and/or with an epitope of a protein comprising a cross-β structure, said molecule produced according to a method as described above.

In one preferred embodiment a composition according to the invention comprises a functional part, derivative and/or analogue of at least one IgIV molecule comprising an affinity region capable of interacting with an epitope of a cross-β structure and/or with an epitope of a protein comprising a cross-β structure. A functional part of an IgIV molecule is defined as a compound which has the same immunological binding properties in kind, not necessarily in amount. Said functional part is capable of binding a cross-β structure and/or protein comprising a cross-β structure, albeit not necessarily to the same extent as said IgIV molecule. A functional derivative of an IgIV molecule is defined as an IgIV molecule which has been altered such that the capability of binding a cross-β structure and/or a protein comprising a cross-β structure of the resulting compound is essentially the same in kind, not necessarily in amount. A derivative is provided in many ways, for instance through conservative amino acid substitution, whereby an amino acid residue is substituted by another residue with generally similar properties (size, hydrophobicity, etc), such that the overall functioning is likely not to be seriously affected, or even improved.

A person skilled in the art is well able to generate analogous compounds of an IgIV molecule. This can for instance be done through screening of a peptide library. Such an analogue is capable of binding a cross-β structure and/or protein comprising a cross-β structure, albeit not necessarily to the same extent as said IgIV molecule.

A selected IgIV molecule and/or an isolated, synthetic or recombinant molecule comprising an affinity region capable of specifically binding an epitope of a cross-β structure and/or an epitope of a protein comprising a cross-β structure is in one embodiment of the invention used for reacting and binding to cross-β structures and/or proteins comprising cross-β structures *in* vitro. Said molecule is preferably reacted with a sample of body fluid or tissue, food, fluid, or a pharmaceutical composition comprising a cross-β structure and/or a protein comprising a cross-β structure, and bound material is preferably removed. Another application of a molecule according to the invention is reacting and binding to cross-β structures and/or proteins comprising a cross-β structure *in vivo*.

One preferred embodiment provides a composition according to the invention wherein at least one of said molecules further comprises a cross-β structure binding compound. A cross-β structure binding compound is a compound capable of specifically binding a cross-β structure. A cross-β structure binding molecule is capable of serving as an effector molecule by enhancing the capability of a molecule of a composition according to the invention to specifically bind a cross-β structure or a protein comprising a cross-β structure. Enhanced binding of a cross-β structure due to said cross-β structure-binding molecule is for instance desired for enhancing the formation and removal of cross-β structure complexes from the circulation and/or from the body. Alternatively, or additionally, local accumulation of cross-β structures such as present in amyloid plaques is diminished.

Non-limiting examples of cross-β structure binding molecules are a finger domain (also referred to as fibronectin type I domain) of tissue-type plasminogen activator (tPA), hepatocyte growth factor activator (HGFA), factor XII, or fibronectin, or members of the multiligand receptor family such as receptor for advanced glycation end-products (RAGE), or low density lipoprotein receptor related protein (LRP) or CD36. Such a cross-β structures binding molecule may even be a non-proteinaceous molecule, such as for example a dye (Congo red or Thioflavin).

In one embodiment, an effector molecule is provided to an isolated, synthetic and/or recombinant molecule of the invention and/or to a selected IgIV immunoglobulin of the invention. A composition and a collection of IgIV molecules according to the invention, wherein at least one of said molecules further comprises an effector molecule, is therefore also provided. In a preferred embodiment said effector compound is capable of enhancing the complement system and/or the phagocytic system of an animal (preferably a human) in order to enhance removal of (proteins comprising) undesired cross-β structures. Hence, in one preferred embodiment said effector compound comprises a complement activating factor such as for instance, but not limited to, any complement protein, a complement activating cytokine, C reactive protein, serum amyloid P component, Pentraxin-3, an Fc region of immunoglobulins (ligand for C1q), a complement control protein, a molecule capable of enhancing the complement activating activity of complement control proteins, and/or a molecules capable of inhibiting the inhibitory activity of complement control proteins. Non-limiting examples of complement control proteins are Cl-inhibitor, C4 binding protein, factor H, factor I, properdin, S protein, complement receptor type I, membrane cofactor protein, decay accelerating factor, C8 binding protein and CD59. In a further preferred embodiment said effector compound is capable of facilitating breakdown of a cross-β structure and/or a protein comprising a cross-β structure. Another preferred property of said effector compound is a capability of facilitating cellular uptake of a cross-β structure and/or a protein comprising a cross-β structure. One embodiment provides a composition according to the invention, wherein said isolated, synthetic and/or recombinant molecule, or said selected IgIV molecule, comprises an effector compound which is a protease or a cross-β structure-binding part thereof. Said effector is particularly suitable for binding and/or breaking down a cross-β structure and/or an undesired protein comprising a cross-β structure. In a further preferred embodiment said effector compound comprises an immunopotentiating compound in order to enhance an immune response directed against a cross-β structure and/or a protein comprising a cross-β structure. Said immunopotentiating compound preferably comprises a cytokine.

In a further embodiment said effector compound comprises a cross-β structure binding-potentiating factor. This is a factor capable of enhancing the capability of a molecule according to the invention of binding a cross-β structure and/or binding a protein comprising a cross-β structure. Non-limiting examples of such factors are Thioflavin T and Thioflavin S (See for instance example 4).

In a further embodiment said effector compound comprises a clearance signal that aids in removal of the resulting complex after a molecule and/or IgIV molecule of the invention has bound a cross-β structure and/or a protein comprising a cross-β structure. Clearance signals are well known in the art. A preferred example of a clearance signal is at least part of an Fc region, more preferably an Fcγ region capable of interacting with an Fc receptor (preferably with an FcγIIb receptor). Said clearance signal is capable of enhancing removal of a complex comprising a molecule according to the invention bound to a cross-β structure or to a protein comprising a cross-β structure from the circulation and/or from the body of an animal (preferably a human).

Activation of the complement system results in a cascade of reactions, including inflammation, cell destruction and tissue damage. In some circumstances it is desired to counteract the complement system in order to dampen adverse side effects. Non-limiting examples of such circumstances are situations with excessive and/or uncontrolled activation of the complement system or (sustained) activation of the complement system without a properly functioning negative feedback mechanism or overstimulation of the complement system, for instance due to sustained and/or overexpressed levels of activators, like for example during inflammation, amyloidoses and/or rheumatoid arthritis. In one embodiment an effector compound is therefore used that is an inflammation suppressive compound, preferably a complement inhibiting factor such as for instance an immunoglobulin or a compound capable of at least partly inhibiting or blocking important functioning of complement proteins and/or capable of at least partly inhibiting or blocking important functioning of any protein or compound that comprises complement system stimulatory capacities. Non-limiting examples of complement inhibiting factors are soluble TNF receptor, IL-1 receptor antagonists and antiinflammatory cytokines.

In yet another embodiment said effector compound comprises an opsonizing compound. Additionally, or alternatively, said isolated, synthetic and/or recombinant molecule of the invention is itself an opsonizing compound. Opsonizing is defined herein as a process of inducing and/or enhancing phagocytosis of a substance by phagocytes such as macrophages, polymorphonuclear cells and the like. Some substances are capable of withstanding and/or escaping phagocytosis, for instance due to the nature of their surface. In such cases, phagocytosis is preferably induced and/or enhanced by opsonizing binding compounds, that, once attached to a substance, facilitate the uptake of said substance by phagocytes such as macrophages and polymorphonuclear cells and the like.

In one embodiment it is determined whether a selected IgIV molecule and/or an isolated, synthetic and/or recombinant molecule according to the invention has an opsonizing capacity, using phagocytic cells. According to this embodiment, once an enriched selection of IgIV molecules according to the invention has been provided, said collection is preferably incubated with a cross-β structure and/or a protein comprising a cross-β structure, where after complexes of IgIV molecules bound to a cross-β structure and/or a protein comprising a cross-β structure are subsequently contacted with a phagocytic cell in order to determine which IgIV molecules are capable of inducing and/or enhancing phagocytosis of said cross-β structure and/or protein comprising a cross-β structure. It is of course also possible to perform the same kind of test with isolated, synthetic and/or recombinant molecules according to the invention. Further provided is therefore a method for selecting from a collection of IgIV molecules according to the invention, or from a composition according to the invention, a molecule comprising an affinity region which is capable, upon interacting with an epitope of a cross-β structure and/or upon interacting with an epitope of a protein comprising a cross-β structure, of inducing opsonization of said cross-β structure and/or a protein comprising a cross-β structure by a phagocytic cell, said method comprising:
- contacting a collection of IgIV molecules according to the invention, and/or a composition according to the invention, with a cross-β structure and/or with a protein comprising a cross-β structure;
- contacting any complex comprising a cross-β structure and/or a protein comprising a cross-β structure, bound to an IgIV molecule and/or to an isolated, synthetic and/or recombinant molecule, with a phagocytic cell; and
- collecting an IgIV molecule and/or isolated, synthetic and/or recombinant molecule that is capable of inducing or enhancing phagocytosis, by a phagocytic cell, of said a cross-β structure and/or a protein comprising a cross-β structure.

Said test is preferably performed *in vitro*. Selected IgIV molecules and/or isolated, synthetic and/or recombinant molecules capable of inducing or enhancing phagocytosis are preferably used in order to induce and/or enhance opsonization of cross-β structures and/or proteins comprising a cross-β structure that are capable of withstanding and/or escaping phagocytosis. Such cross-β structures and/or proteins comprising a cross-β structure capable of withstanding and/or escaping phagocytosis for instance occur in disease states in which molecules capable of inducing or enhancing phagocytosis are absent or present at reduced (functional) levels, like for example in AIDS, SCIDS and a-gammaglobulinaemia, and for instance in disease states in which formation of cross-β structures and/or proteins comprising a cross-β structure is increased like for example in TSE, amyloidoses, diabetes, thrombosis and inflammation.

As described before, cross-β structures in proteins are often related to, and/or associated with, a risk and/or presence of disease, such as for instance Huntington's disease, amyloidosis type disease, atherosclerosis, diabetes, bleeding, thrombosis, cancer, sepsis and other inflammatory diseases, rheumatoid arthritis, transmissible spongiform encephalopathies such as Creutzfeldt-Jakob disease, Multiple Sclerosis, auto-immune diseases, diseases associated with loss of memory such as Alzheimer's disease, Parkinson's disease and other neuronal diseases (epilepsy), encephalopathy and systemic amyloidoses. An enriched collection of IgIV molecules according to the invention and a collection of isolated, synthetic and/or recombinant molecules according to the invention, being capable of specifically binding cross-β structures and/or proteins comprising a cross-β structure, are particularly suitable for at least in part preventing and/or treating such cross-β structure related and/or associated diseases. One embodiment therefore provides a collection of IgIV molecules according to the invention and/or a composition according to the invention for use as a medicament and/or prophylactic agent. The invention furthermore provides a use of a collection of IgIV molecules and/or a composition according to the invention for the preparation of a medicament and/or prophylactic agent. Said medicament and/or prophylactic agent is particularly suitable for at least in part preventing, treating and/or stabilizing diseases that are related to and/or associated with occurrence of cross-β structures, blood coagulation disorders, inflammation, and/or an infection by a microbe, pathogen, bacterium, parasite and/or virus. Further provided is therefore a use of a collection of IgIV molecules according to the invention and/or a composition according to the invention for the manufacture of a medicament for at least partial prevention and/or treatment of a cross-β structure related and/or associated disease, a blood coagulation disorder, inflammation and/or a microbial/pathogen/parasite/bacterial/viral infection. A method for at least partial prevention and/or treatment of a cross-β structure related and/or associated disease, a blood coagulation disorder and/or a microbial/pathogen/parasite/bacterial/viral infection in an individual, comprising administering a collection of IgIV molecules according to the invention and/or a composition according to the invention to said individual, is also herewith provided.

In one preferred embodiment said microbial/pathogen/parasite/bacterial/viral infection comprises an opportunistic infection. This is an infection by an organism such as for instance a pathogen and/or virus that does not ordinarily cause disease but that, under certain circumstances (such as an impaired immune system), becomes pathogenic. An impaired immune system is for instance caused by medication such as chemotherapy. In a particularly preferred embodiment said microbial/pathogen/parasite/bacterial/viral infection comprises an HIV-related opportunistic infection. Since opportunistic infections are the major cause of death in HIV patients, it is highly desired to provide medicaments and/or prophylactic agents against such infections. Many opportunistic infections involve the presence of a cross-β structure. For instance, amyloid structures occur on the surface of microbial organisms like fungi, yeast and bacteria. Said amyloid-like structures are generally called hydrophobins on fungi, chaplins on gram-positive bacteria, and curli or tafi or aggregative fimbriae on gram-negative bacteria. Since an enriched collection of IgIV molecules according to the invention and a collection of isolated, synthetic and/or recombinant molecules according to the invention are particularly suitable for binding such cross-β structures and/or proteins comprising a cross-β structure, said collections of the invention are particularly suitable for counteracting and/or at least in part preventing HIV-related opportunistic infections. The invention therefore provides a method for at least partial prevention and or treatment of an HIV-related opportunistic infection in an individual, comprising administering a collection of IgIV molecules according to the invention and/or a composition according to the invention to said individual.

A composition comprising a collection of IgIV molecules according to the invention and/or a composition according to the invention and a suitable carrier, diluent and/or excipient is also herewith provided. Said composition preferably comprises a pharmaceutical composition. In order to be able to administer a medicament according to the present invention to a patient in need of treatment, said medicament must fulfil the needs for a pharmaceutically acceptable formulation. This means that a medicament according to the invention comprises an enriched collection of IgIV molecules according to the invention and/or a collection of isolated, synthetic and/or recombinant molecules according to the invention which are of pharmaceutical grade, physiologically acceptable and tested for extraneous agents. A pharmaceutical composition comprising an enriched collection of IgIV molecules according to the invention and/or a collection of isolated, synthetic and/or recombinant molecules according to the invention and a pharmaceutically acceptable carrier, diluent and/or excipient is also herewith provided. Preferably, said composition comprises a cross-β structure-binding compound in order to enhance interaction of said pharmaceutical composition with a cross-β structure and/or with a protein comprising a cross-β structure. Therefore, the invention provides a composition according to the invention further comprising a cross-β structure-binding compound. In a further preferred embodiment of the invention, binding of a composition according to the invention to a cross-β structure and/or to a protein comprising a cross-β structure is further enhanced or potentiated by the addition of a compound that is known for its cross-β structure-binding-potentiating characteristics, such as for example dye molecules such as Thioflavin T or Thioflavin S. Therefore, the present invention discloses a composition according to the invention further comprising a cross-β structure-binding-potentiating compound.

In another preferred embodiment, removal of cross-β structures and/or proteins comprising a cross-β structure from a body is enhanced by adding to a composition according to the invention complement potentiating signals capable of enhancing complement activation. Therefore, the invention provides a composition according the invention, further comprising a complement potentiating compound.

Since activation of the complement system results in a cascade of reactions, including inflammation, cell destruction and tissue damage, it is sometimes desired to at least in part counteract complement activation. In some cases, activation of the complement system in relation to the clearance of cross-β structures is itself causing illness. In such cases, a composition according to the invention preferably further comprises a complement inhibiting compound. In one embodiment a composition according to the invention comprises an inflammation suppressive compound.

The present invention furthermore provides means and methods for increasing extracellular protein degradation and/or protein clearance in an individual. In a natural situation, the formation of cross-β structures initiates and/or participates in a physiological cascade of events, dealing with removal of unwanted molecules, such as for instance misfolded proteins, apoptotic cells or even pathogens. This pathway regulates the removal of unwanted biomolecules during several processes, including protein misfolding during synthesis in the endoplasmic reticulum, fibrinolysis, formation of neuronal synaptic networks, clearance of used, unwanted and/or destroyed (denatured) proteins, induction of apoptosis and clearance of apoptotic cells, necrotic cells, aged cells and/or pathogens. Since a collection of IgIV molecules according to the invention and a composition according to the invention are particularly suitable for binding cross-β structures and proteins comprising cross-β structures, extracellular protein degradation and/or protein clearance is increased. Further provided is therefore a method for increasing extracellular protein degradation and/or protein clearance in an individual, comprising administering a collection of IgIV molecules according to the invention and/or a composition according to the invention to said individual.

By binding and removing cross-β structures and proteins comprising cross-β structures, a collection of IgIV molecules according to the invention and a composition according to the invention are capable of at least in part counteracting cross-β structure mediated effects in an individual. Further provided is therefore a method for at least in part inhibiting cross-β structure mediated effects in an individual, comprising administering an effective amount of a collection of IgIV molecules according to the invention and/or a composition according to the invention to an individual.

In a preferred embodiment, a collection of IgIV molecules according to the invention and/or a composition according to the invention is used in order to inhibit platelet aggregation that is induced by proteins comprising a cross-β structure. An example of such use is shown in Example 2. Therefore, the invention provides a use of a collection of IgIV molecules according to the invention and/or a composition according to the invention for inhibiting protein-induced blood-platelet aggregation.

In another preferred embodiment, a collection of IgIV molecules according to the invention and/or a composition according to the invention is used in order to compete binding of the serine protease tissue type plasminogen activator (tPA) to a cross-β structure and/or to a protein comprising a cross-β structure. tPA induces the formation of plasmin through cleavage of plasminogen. Plasmin cleaves fibrin and this occurs during lysis of a blood clot. Although not essential for fibrinolysis in mice, tPA has been recognized for its role in fibrinolysis for a long time. Activation of plasminogen by tPA is stimulated by fibrin or fibrin fragments, but not by its precursor, fibrinogen. tPA is a cross-β structure binding protein, a multiligand receptor and a member of the cross-β structure pathway. tPA mediates a cross-β structure induced cell dysfunction and/or cell toxicity. tPA mediates at least in part cell dysfunction and/or toxicity through activation of plasminogen. The plasminogen dependent effects are inhibited with a collection of IgIV molecules according to the invention and/or a composition according to the invention. Excessive or uncontrolled tPA/plasminogen activation during a disease state is treated this way. Non-limiting examples of such disease states are Alzheimer's disease, infections, preeclampsia, angina pectoris, inflammatory and noninflammatory joint diseases, diabetes.

One preferred embodiment provides a use of a collection of IgIV molecules and/or a composition according to the invention for at least partial removal of cross-β structures and/or proteins comprising a cross-β structure from a sample. Removal of cross-β structures and/or proteins comprising a cross-β structure is desired in a variety of applications. For instance, if an individual is suffering from, or at risk of suffering from, a disorder related to and/or associated with the presence of a cross-β structure, removal of such cross-β structure from the body is beneficial in order to counteract such disorder and/or to alleviate adverse side effects. Moreover, it is advantageous to remove cross-β structures and/or proteins comprising a cross-β structure from products intended for (human) consumption in order to at least in part avoid uptake of cross-β structures. One embodiment therefore provides a method for at least partially removing cross-β structures and/or proteins comprising a cross-β structure from a sample, said method comprising contacting a sample with a collection of IgIV molecules according to the invention and/or a composition according to the invention, and removing from said sample any complexes of cross-β structures, and/or proteins comprising a cross-β structure, bound to an IgIV molecule and/or an isolated, synthetic and/or recombinant molecule. Said sample preferably comprises a fluid sample. In one embodiment said fluid comprises a food substance.

In one preferred embodiment said sample comprises a body fluid. This embodiment is particularly suitable for at least in part preventing and/or treating a cross-β structure related and/or associated disorder of an animal, preferably of a human individual. In one preferred embodiment extracorporeal dialysis is applied. For example, a patient suffering from a cross-β structure related and/or associated disorder is subjected to dialysis of his blood. A collection of IgIV molecules and/or a composition according to the invention is for instance coupled to a carrier or support and/or to the inside of a tube used for dialysis. This way, cross-β structures and proteins comprising a cross-β structure will be removed from the blood stream of said patient, thereby at least in part relieving said patient of negative effects related to, and/or associated with, said cross-β structures and/or proteins comprising a cross-β structure. As another example, such use is applied in haemodialysis of kidney patients. A separation device for carrying out a method according to the invention is also provided. One embodiment thus provides a separation device for carrying out a method according to the invention, said device comprising a system for transporting (circulating) fluids, said system being provided with means for connecting to a flowing fluid, preferably to an individual's circulation, means for entry of fluid into said system and return of fluid from said system, preferably to an individual's circulation, said system further comprising a solid phase, said solid phase comprising a collection of IgIV molecules according to the invention and/or a composition according to the invention. Said separation device preferably comprises a dialysis apparatus.

Another preferred embodiment provides a use of a collection of IgIV molecules and a composition according to the invention for at least partial removal of cross-β structures and/or proteins comprising a cross-β structure from a pharmaceutical or any of its constituents. Important categories of nowadays pharmaceutical compositions comprising a protein or a proteinaceous compound as an active substance include, but are not limited to hormones, enzymes, vaccines and antigens, cytokines and antibodies. In addition to the above-mentioned proteinaceous pharmaceutical compositions, a large number of pharmaceutical compositions are manufactured with the help of a production and/or purification step comprising proteins. For example, many pharmaceutical compositions comprise one or more proteins as a stabilizing agent. Health problems related to the use of pharmaceutical compositions are for example related to the fields of haematology, fibrinolysis and immunology. An incomplete list of observed side-effects after administration of pharmaceutical compositions comprises for example fever, anaphylactic responses, (auto)immune responses, disturbance of haemostasis, inflammation, fibrinolytic problems, including sepsis and disseminated intravascular coagulation (DIC), which can be fatal. Said side effects are for instance caused by either an alteration of a protein or a proteinaceous compound present in said pharmaceutical composition, or by added diluents or carrier substances of said pharmaceutical composition. Alteration of a proteinaceous compound of a pharmaceutical composition comprises for example denaturation, multimerization, proteolysis, acetylation, glycation, oxidation, unfolding or misfolding of proteins. Unfolding or misfolding of initially properly folded native proteins leads to the formation of toxic structures in said proteins. Toxic structures of pharmaceutical compositions often comprise cross-β structures. Said toxic structures are at least in part removed with a collection of IgIV molecules and/or a composition according to the invention.

Provided is therefore a method for removing a cross-β structure and/or protein comprising a cross-β structure from a pharmaceutical composition or any of its constituents comprising a protein, said method comprising:
- contacting said pharmaceutical composition or any of its constituents comprising a protein with a collection of IgIV molecules according to the invention and/or with a composition according to the invention;
- allowing binding of said cross-β structure and/or protein comprising a cross-β structure to said collection of IgIV molecules and/or composition; and
- separating bound cross-β structure and/or bound protein comprising a cross-β structure from said pharmaceutical composition or any of its constituents comprising a protein.

By removing a cross-β structure and/or a protein comprising a cross-β structure from a pharmaceutical composition, undesired side effects are at least in part decreased and/or prevented. Also provided is therefore a method for decreasing and/or preventing undesired side effects of a pharmaceutical composition and/or increasing the specific activity per gram protein, said method comprising removing an unfolded protein, an unfolded peptide, a misfolded protein, a denatured protein, an aggregated protein, an aggregated peptide, a multimerized protein and/or a multimerized peptide, comprising a cross-β structure, from said pharmaceutical composition or any of its constituents, using a method according to the invention.

A pharmaceutical composition or any of its constituents comprising a protein, obtainable by a method according to the invention is also herewith provided. Said pharmaceutical composition involves a reduced risk of undesired side effects as compared to untreated pharmaceutical compositions.

In one embodiment a cross-β structure and/or protein comprising a cross-β structure is removed from a sample using a collection of IgIV molecules and/or a composition of isolated, synthetic and/or recombinant molecules according to the invention, wherein said collection and/or composition is bound to a solid support. This provides the advantage that a continuous process has become possible, wherein said solid support is incubated with a sample. Subsequently, said sample and said solid support are easily separated from each other, said solid support comprising cross-β structures and/or proteins comprising a cross-β structure that are (indirectly) bound, while the resulting sample has a lowered concentration of cross-β structures and/or proteins comprising a cross-β structure.

In yet another embodiment, a selected IgIV immunoglobulin and/or an isolated, synthetic and/or recombinant molecule according to the invention is used to make a diagnostic kit. Said diagnostic kit is particularly suitable for diagnosis of a disease that is related to, and/or associated with, the presence of cross-β structures. Said kit preferably comprises at least one affinity region of a collection of IgIV molecules according to the invention, and/or at least one affinity region of a composition according to the invention, capable of interacting with a cross-β structure and/or with a protein comprising a cross-β-structure, and a way of visualization of an interaction of said cross-β structure and/or said protein with said affinity region.

With such diagnostic kit, not only diseases that are generally related to and/or associated with the presence of cross-β structures are diagnosed, but also a more defined diagnosis is possible, dependent of the specificity of the affinity regions in the kit. A diagnostic kit capable of specifically diagnosing one kind of disorder is for instance generated by providing said kit with affinity regions which are capable of specifically binding a given cross-β structure and/or a given protein comprising a cross-β structure that is specific for said one kind of disorder, such as for example proteins related to rheumatoid arthritis, SLE or other autoimmune diseases, or inflammatory reactions. Therefore, in one embodiment, the invention provides a diagnostic kit as described above, wherein said cross-β structure is a disease-related cross-β structure.

Since cross-β structures and proteins comprising a cross-β structure are effectively bound to a collection of IgIV molecules according to the invention and/or to a composition according to the invention, they are effectively separated and/or isolated from a sample and/or an animal's or human's body and subsequently identified. In yet another embodiment therefore, a selected IgIV immunoglobulin and/or an isolated, synthetic and/or recombinant molecule according to the invention is used to isolate cross-β structures and/or proteins comprising a cross-β structure. Preferably, cross-β structures and/or proteins comprising a cross-β structure present in a body fluid, like for example blood, serum, plasma, cerebrospinal fluid, synovial fluid, sputum and/or urine, is identified. For instance, the presence and/or identity of a cross-β structure, and/or protein comprising a cross-β structure, of healthy individuals is compared with the presence and/or identity of a cross-β structure, and/or protein comprising a cross-β structure, from individuals with a disease related to and/or associated with a cross-β structure and/or a protein comprising a cross-β structure. The identity and the relative concentration of a cross-β structure and/or protein comprising a cross-β structure is determined using any method known to a person skilled in the art, like for example, but not limited to, 2D gel electrophoresis and/or mass-spectrometric analyses. The results of a sample originating from a healthy individual and a sample originating from a patient are preferably compared. In this way, information is obtained, for instance about the identity and/or susceptibility of proteins prone to misfold and adopt cross-β structure conformation during defined disease states. This obtained information subsequently serves as a diagnostic tool, for instance to monitor disease state, to monitor effectiveness of therapy, to monitor occurrence of disease, and provides valuable leads for development of therapeutics targeted at cross-β structures and/or protein(s) comprising a cross-β structure which are preferably specific for a defined disease.

The invention therefore provides a method for determination of the identity of a cross-β structure or a protein comprising a cross-β structure in a sample comprising a protein, said method comprising:
- contacting said sample with a collection of IgIV molecules according to the invention, and/or a composition according to the invention, resulting in bound cross-β structures and/or bound protein(s) comprising a cross-β structure, and
- identifying a bound cross-β structure and/or a bound protein comprising a cross-β structure. Said bound cross-β structure and/or bound protein comprising a cross-β structure is preferably identified by analyzing at least part of the amino acid sequence of said cross-β structure and/or protein using any method known in the art. Said sample preferably comprises an aqueous solution, more preferably a body fluid. In one preferred embodiment body fluids originating from healthy individuals (preferably humans) and body fluids originating from individuals suffering from, or suspected to suffer from, a disease related to and/or associated with the presence of a cross-β structure are used in order to compare a healthy state with a diseased state (or a state wherein the risk of disease is enhanced).

Because the present invention provides a way of selecting from a collection of IgIV those immunoglobulins that have affinity regions capable of interacting with a cross-β structure and/or with a protein comprising a cross-β structure, a skilled person is now also capable of using said selected IgIV, and/or isolated, synthetic and/or recombinant molecules according to the invention, in order to determine whether a protein or peptide comprising a cross-β structure is present in a sample. Provided is therefore a method for determining whether a protein and/or peptide comprising a cross-β structure is present in an aqueous solution comprising a protein, said method comprising:
- contacting said aqueous solution with a collection of IgIV molecules according to the invention, and/or a composition according to the invention, and
- detecting whether bound protein and/or peptide comprising a cross-β structure is present. Said protein and/or peptide is preferably detected in an aqueous solution by contacting said aqueous solution with a collection and/or composition of the invention and detecting bound peptides and/or proteins. Provided is thus a method for detecting a protein and/or peptide comprising a cross-β structure in an aqueous solution comprising a protein, said method comprising contacting said aqueous solution with a collection of IgIV molecules according to the invention, and/or a composition according to the invention, resulting in a bound protein and/or peptide comprising a cross-β structure, and detecting bound protein and/or peptide comprising a cross-β structure. Binding of said collection and/or composition of the invention to a cross-β structure is preferably detected by means of a visualization reaction as for example by fluorescent staining or an enzymatic or colorimetric detection, or by any other visualization system available to a skilled person.

Said aqueous solution preferably comprises a detergent, a food product, a food supplement, a cell culture medium, a commercially available protein solution used for research purposes, blood, a blood product, a body fluid like for example urine, cerebrospinal fluid, synovial fluid, lymph fluid and/or sputum, a cosmetic product, a cell, a pharmaceutical composition or any of its constituents comprising a protein, or a combination of any of these.

A use of a collection of IgIV molecules according to the invention, and/or a composition according to the invention, for determining the presence of accumulated deposited misfolded protein with a cross-β structure, is also herewith provided. Preferably, the presence of a misfolded protein involved in a conformational disease is detected. A conformational disease is defined as a disease that is caused by, related to and/or associated with misfolding of proteins and/or conformational change of proteins.

One embodiment furthermore comprises detection of the amount of a cross-β structure and/or a protein comprising a cross-β structure in a composition. This is for instance done in order to determine the course of a disease. Further provided is therefore a method for determining the amount of a cross-β structure and/or protein comprising a cross-β structure in a composition, preferably in a medicament and/or vaccine, comprising contacting said composition with a collection of IgIV molecules according to the invention, and/or with a composition according to the invention, and relating the amount of bound cross-β structures and/or proteins comprising a cross-β structure to the amount of cross-β structures and/or proteins comprising a cross-β structure present in said composition.

Since misfolded proteins comprising a cross-β structure are effectively bound to a collection of IgIV molecules according to the invention and to a composition according to the invention, they are effectively removed from a sample and/or an animal's body (preferably a human's body). This way, accumulation of misfolded proteins is diminished. Further provided is therefore a use of a collection of IgIV molecules according to the invention, and/or a composition according to the invention, for diminishing accumulation of misfolded protein comprising a cross-β structure. Said misfolded protein comprising a cross-β structure is preferably involved in a conformational disease. Diminishing accumulation of such proteins results in alleviation of symptoms of said conformational disease and/or at least partial treatment and/or prevention of the course of disease. Said conformational disease preferably comprises an amyloidosis type disease, atherosclerosis, diabetes, bleeding, thrombosis, cancer, sepsis and other inflammatory diseases, rheumatoid arthritis, transmissible spongiform encephalopathies, Multiple Sclerosis, auto-immune diseases, disease associated with loss of memory or Parkinson's disease and other neuronal diseases (epilepsy), encephalopathy, and/or rheuma.

Coagulation of blood and blood platelet clot formation also involves the presence of cross-β structures. Examples of the role of (misfolded) protein comprising cross-β structures are activation of platelets and induction of platelet aggregation and agglutination, activation of endothelium resulting in tissue factor expression and exposure to blood, resulting in blood coagulation, and activation of the contact system of blood coagulation via activation of factor XII. In addition, during blood coagulation fibrin polymers with cross-β structure conformation are formed. The cross-β structure building block of a fibrin network subsequently serves as the binding site for tPA to localize tPA at the site where fibrinolytic activity is required. Since a collection and composition according to the invention are capable of specifically binding and/or removing cross-β structures and proteins comprising cross-β structures, said collection and composition are particularly suitable for interfering in coagulation of blood and/or clot formation and/or activation of tissue factor. Further provided is therefore a method for interfering in coagulation of blood and/or clot formation comprising providing to blood a collection of IgIV molecules according to the invention, and/or a composition according to the invention.

Also provided is a method for determining a difference in the cross-β structure content of a protein in a reference sample compared to the cross-β structure content of said protein in a test sample, wherein said test sample has been subjected to a treatment that is expected to have an effect on the cross-β structure content of said protein, the method comprising:
- determining in a reference sample the cross-β structure content of a protein using a collection of IgIV molecules according to the invention and/or a composition according to the invention;
- subjecting said protein to a treatment that is expected to have an effect on the cross-β structure content of said protein, thus obtaining a test sample;
- determining in the obtained test sample the cross-β structure content of said protein using a collection of IgIV molecules according to the invention, and/or a composition according to the invention; and
- determining whether the cross-β structure content of said protein in said reference sample is significantly different from the cross-β structure content of said protein in said test sample.

This embodiment is particularly suitable for determining whether a certain circumstance and/or treatment has an effect on the cross-β structure content of a protein. Once this has been determined, it is possible to select a circumstance and/or treatment that has a low capability of inducing and/or enhancing cross-β structure conformation. Of course, it is also possible to choose a circumstance and/or treatment that is well capable of inducing and/or enhancing cross-β structure conformation, depending on a particular application.

The invention is further explained by the following examples, without being restricted to them.

### EXAMPLES

### Materials & Methods

### Materials

Human broad spectrum immunoglobulin G (IgG) antibodies, referred to as 'intravenous Ig' ('IVIg' or 'IgIV'), 'gammaglobulin', 'intravenous immune globulin', 'intravenous immunoglobulin' or otherwise, were obtained from the local University Medical Center Utrecht pharmacy department. Octagam from Octapharma (Octapharma International Services N.V., Brussel, Belgium; dosage 2.5 gr. in 50 ml, lot 4270568431, exp. 05-2006, hereinafter referred to as IgIV 'manufacturer I', or IgIV (I) or IgIV-I) and Hyland Immuno Gammagard S/D IVIg from Baxter (Baxter B.V., Utrecht, The Netherlands; dosage 5 gr. with 96 ml reconstitution solution, lot LE08E044AL, exp. 04-2007, hereinafter referred to as IgIV 'manufacturer II', IgIV (II) or IgIV-II) were used. Gammagard was reconstituted under sterile conditions by adding the supplied 96 ml H₂O and leaving the solution for 30' on a roller device at room temperature (final IgG concentration 52 mg/ml. A clear solution was obtained without foam formation. The reconstituted solution was aliquoted and stored at -20°C. After reconstitution, the Gammagard solution contains 0.06 gr. pasteurized human albumin, 0.45 gr. glycine, 0.175 gr. NaCl, 0.43 gr. glucose-monohydrate and 0.04 gr. polyethylene glycol 3,350. Octagam is supplied as a ready-to-use solution comprising 50 mg/ml IgIV. Other components are 100 mg/ml maltose and less than 5 µg/ml Triton X-100 and less than 1 µg/ml tri-n-butyl phosphate. It is stored at 4°C. According to the manufacturer, Octagam mainly consists of IgG's (≥95%), with a minor IgA fraction (≤5%). The distribution over the four IgG isotypes is: IgG1, 62.6%; IgG2, 30.1%; IgG3, 6.1%; IgG4, 1.2%. Gammagard and Octagam are used at room temperature. Solutions were kept at room temperature for at least 30' before use. Frozen aliquots of Gammagard were first quickly thawed to approximately 0°C and then left at room temperature. A third source of human immunoglobulins was normal pooled citrated plasma of approximately 40 apparently healthy donors, prepared at the University Medical Center Utrecht. This plasma was mixed directly after the blood was drawn, and directly aliquoted and frozen at -80°C. Before use, an aliquot was thawed for 10' in a 37°C-water bath and kept at room temperature for 30'. The plasma was mixed by swirling and/or by resuspending with a pipette; vortexing was avoided, as was done with the IgIV preparations and all other protein solutions used.

For ELISA's Microlon high-binding plates (Greiner Bio-One GmbH, Frickenhausen, Germany; catalogue number 655092, lot 05130103, exp. 03-2009) were used. Antibodies used were goat anti-human IgG-alkaline phosphatase (Biosource Int., Camarillo, CA, USA; catalogue number AHI0305, lot 7602), goat anti-human IgM-alkaline phosphatase (Biosource Int.; catalogue number AHI0605, lot 3903), peroxidase-conjugated rabbit antimouse immunoglobulins (RAMPO, catalogue number P0260, DAKOCytomation, Glostrup, Denmark), peroxidase-coupled swine anti-rabbit immunoglobulins (SWARPO, catalogue number P0217, DAKOCytomation), rabbit polyclonal anti-human albumin antibody A-0001 (DAKOCytomation), rabbit polyclonal anti-human haemoglobin antibody A-0118 (DAKOCytomation; lot 122(021)), mouse monoclonal anti-human amyloid-β antibody M0872 (DAKOCytomation; clone 6F/3D, lot 00003503, exp. 08-2006), rabbit polyclonal anti-human fibrinogen antibody A0080 (DAKOCytomation; lot 097(701), exp. 08-2006) and murine monoclonal hybridoma anti-glucose-6-phosphate glycated human fibronectin antibody 4B5 (lot 2, code 100901BB, ref. (Bouma et al., 2003)). In ELISA's binding of alkaline phosphatase conjugated antibodies was assessed using p-nitrophenyl phosphate disodium 6*H₂O (Sigma-Aldrich, St. Louis, MO, USA; Phosphatase substrate catalogue number 104, lot 120K6008), and binding of peroxidase-conjugated antibodies was assessed using 1,2-phenylenediamine ('OPD', Merck, Darmstadt, Germany; catalogue number 1.07243.0050, lot L937543-844).

Inhibition studies using an ELISA set-up were performed using concentration series of Congo red (Aldrich, Milwaukee, WI, USA; catalogue number 86,095-6), Thioflavin T (Sigma, St. Louis, MO, USA; catalogue number T3516, lot 80K3444), Thioflavin S (Sigma; catalogue number T1892), tissue-type plasminogen activator (tPA, Actilyse, Boehringer-Ingelheim, Alkmaar, The Netherlands), or a truncated form of tPA (K2P tPA, Rapilysin, Boehringer-Ingelheim, Alkmaar, The Netherlands) lacking three amino-terminal domains including the fibronectin type I domain, or alternatively designated as finger (F) domain.

Antigens used in IgIV binding ELISA's were synthetic human fibrin peptide 148-KRLEVDIDIGIRS-160 *(SEQ-ID* 1), with a K157G mutation, synthetic human amyloid-β peptide 1-DAEFRHDSGYEVHHQKLVFFAEDVGSNKGAIIGLMVGGVV-40 *(SEQ-ID 2)* (Aβ(1-40), synthetic human Aβ(1-40)E22Q Dutch type 1-DAEFRHDSGYEVHHQKLVFFAQDVGSNKGAIIGLMVGGVV-40 *(SEQ-ID 3)* (Peptide facility, Dutch Cancer Institute, Amsterdam, the Netherlands), bovine serum albumin (BSA, fraction V, catalogue number A-7906, initial fractionation by heat shock, purity ≥ 98% (electrophoresis), remainder mostly globulins, Sigma-Aldrich, St. Louis, MO, USA), human haemoglobin (Hb, Sigma-Aldrich; catalogue number H7379), and their advanced glycated endproducts-modified counterparts BSA-AGE and Hb-AGE (see below).

### Methods

### Glycation of proteins

Glycation of albumin and Hb was performed as follows. For preparation of BSA-AGE, 100 mg ml⁻¹ of albumin was incubated with phosphate-buffered saline (PBS, 140 mM sodium chloride, 2.7 mM potassium chloride, 10 mM disodium hydrogen phosphate, 1.8 mM potassium di-hydrogen phosphate, pH 7.3) containing 1 M of D-glucose-6-phosphate disodium salt hydrate (anhydrous) (g6p, ICN, Aurora, Ohio, USA) and 0.05% m/v NaN₃, at 37°C in the dark. The solution was glycated for 70 weeks. Human Hb at 10 mg/ml was incubated for 75 weeks at 37°C with PBS containing 1 M of g6p and 0.05% m/v of NaN₃. After incubations, albumin and Hb solutions were extensively dialysed against distilled water and, subsequently, aliquoted and stored at-20°C. Protein concentrations were determined with Advanced protein-assay reagent ADV01 (Cytoskeleton, Denver, CO, USA).

### Preparation of heat-denatured proteins

Heat denatured misfolded proteins were prepared as follows. One mg/ml of Endostatin (recombinantly produced collagen XVIII fragment, EntreMed, Inc., Rockville, MD; solution), BSA (Sigma-Aldrich; lyophilized, catalogue number A7906), murine serum albumin (MSA, Calbiochem, EMD Biosciences, Inc., San Diego, CA; lyophilized, catalogue number 126674), hen egg-white lysozyme (ICN, Irvine, CA, USA; lyophilized, catalogue number 100831), human glucagon (Glucagen, Novo Nordisk, Copenhagen, Denmark; lyophilized, catalogue number PW60126), purified chicken ovalbumin (OVA, Sigma; catalogue number A7641, lot 071k7094) or human B2-glycoprotein I (β2gpi, purified in-house, from fresh plasma, ref. (Horbach et al., 1996)) in 67 mM NaPi buffer pH 7.0, 100 mM NaCl, was heated for five cycles in PCR cups in a PTC-200 thermal cycler (MJ Research, Inc., Waltham, MA, USA). In each cycle, proteins were heated from 30 to 85°C at a rate of 5°C/min. In addition, Endostatin, MSA, ovalbumin and lysozyme were heat-denatured at 1 mg/ml in a similar way, using only one heat incubation cycle. Endostatin at 7.9 mg/ml was diluted in H₂O to 1 mg/ml, MSA and ovalbumin at 1 mg/ml were in PBS pH 7.4, lysozyme was dissolved in PBS with 10 µM HCl added, 1 mg/ml concentration. Control proteins are not subjected to the thermal cycling procedure. To confirm misfolding of the proteins into amyloid-like structures, enhancement of Thioflavin T (ThT) was assessed with heat-treated proteins as well as with control proteins. Fluorescence of ThT - amyloid-like protein/peptide adducts was measured as follows. Solutions of 25 µg ml⁻¹ of protein or peptide preparations were prepared in 50 mM glycine buffer pH 9.0 with 25 µM ThT. Fluorescence was measured at 485 nm upon excitation at 435 nm. Background signals from buffer, buffer with ThT and protein/peptide solution without ThT were subtracted from corresponding measurements with protein solution incubated with ThT. Regularly, fluorescence of Aβ was used as a positive control, and fluorescence of synthetic human fibrin fragment FP10 (148-KRLEVDIDIK-157 *(SEQ-ID 4);* Peptide facility, Dutch Cancer Institute, Amsterdam, the Netherlands), a non-amyloid fibrin fragment(Kranenburg et al., 2002), and buffer was used as a negative control. Fluorescence was measured in triplicate on a Hitachi F-4500 fluorescence spectrophotometer (Hitachi, Ltd., Tokyo, Japan). Alternatively, Congo red fluorescence was analyzed in a similar way. Now, excitation and emission wavelengths were 550 and 590 nm. Again, 25 µg/ml of tester proteins was analyzed, in 25 µM Congo red solutions.

Alternatively, a heat denatured amyloid peptide was prepared as follows. Human fibrin peptide NH₂-IDIKIR-COOH *(SEQ-ID 6,* FP6) was dissolved at approximately 10 mg/ml in a 1:1 volume ratio of 1,1,1,3,3,3-hexafluoro-2-propanol and trifluoro acetic acid. The organic solvents were vaporized under an air stream. FP6 was dissolved in distilled water to a final concentration of 1 mg/ml and kept at 37°C for 72 h. The solution was subsequently stored at room temperature. Presence of crossbeta structure conformation was confirmed by measuring enhancement of fluorescence of amyloid specific dyes ThT and Congo red and by X-ray fiber diffraction analysis (personal communication, L. Kroon-Batenburg, Bijvoet Center for Biomolecular Research, Dept. of Crystal & Structural Chemistry, University of Utrecht, The Netherlands) (data not shown here). Furthermore, the property of FP6 solutions to activate tPA in a plasminogen/plasmin/chromogenic substrate conversion assay was assessed and found to be positive (data shown elsewhere).

### Preparation of yeast prion peptide oligomers with amyloid-like conformation

The peptide fragment NH₂-GNNQQNY-COOH of the yeast prion protein *(SEQ-ID 5*) was purchased from the Peptide Facility of the Netherlands Cancer Institute (H. Hilkmann, NKI-Amsterdam, The Netherlands; lot 5LKB1-2081). Purity of the peptide was analyzed by performing reversed phase HPLC and was ~90%. The peptide was dissolved to final concentrations of 1 and 10 mg/ml in H₂O. The clear solutions were incubated for 72 h at 4°C at a rollerbank or for 5 h at room temperature without motion. Enhancement of Congo red fluorescence was determined as a measure for the presence of amyloid like conformation (see above). In addition, formation of crossbeta structure with this batch of peptide was confirmed with X-ray fiber diffraction analysis using a solution of 10 mg/ml in H₂O (personal communication, L. Kroon-Batenburg, Bijvoet Center for Biomolecular Research, Dept. of Crystal & Structural Chemistry, University of Utrecht, The Netherlands) (data not shown here).

### Preparation of oxidized proteins

Oxidation of proteins was performed using prolonged exposure of proteins in solution to CuSO₄. Proteins used were human normal pooled citrated plasma of apparently healthy persons, formulated Endostatin (EntreMed, Inc., Rockville, MD; 7.9 mg/ml solution), chicken egg-white lysozyme (ICN, catalogue number 100831, lot 98032), human haemoglobin (Sigma-Aldrich, catalogue number H7379, lot 039H7605), human glucagon (Glucagen from NovoNordisk Farma B.V., lot RW 60038), bovine albumin (Sigma-Aldrich, A7906, lot 81K1813), human γ-globulins (Sigma-Aldrich, G4386, lot 21K7600), chicken egg-white ovalbumin (Sigma-Aldrich, A7641, lot 071K7094). Lyophilized proteins were dissolved at 2 mg/ml in PBS, plasma was 40 times diluted and Endostatin was diluted to 2 mg/ml in PBS. NaN₃ stock solution of 2% m/v was added to a final concentration of 0.02%. CuSO₄ stock solution of 1 M in H₂O was added to a final concentration of 10 mM. In control protein solutions H₂O was added instead of CuSO₄. All protein solutions were mixed by swirling, avoiding vortexing. Solutions were kept at 4°C on a rollerbank for 72 h. Enhancement of ThT was measured (see above).

Alternatively, proteins were oxidized by introducing 10 µM CuSO₄ in the solutions. In this way, ovalbumin, albumin, endostatin, lysozyme, γ-globulins all at 2.5 mg/ml and glucagon at 1 mg/ml were incubated for 144 h at 37°C in PBS. In control protein solutions, CuSO₄ was omitted. Thioflavin T fluorescence was measured as a measure for the presence of misfolded proteins with crossbeta structure conformation. Protein solutions that showed enhanced ThT fluorescence were dialyzed against PBS, as well as their non-oxidized controls.

Low density lipoproteins (LDL) were isolated from fresh (<24 h) human plasma that was kept at 10°C, obtained from the Netherlands bloodbank. LDL was isolated essentially as earlier described. Plasma was centrifuged in an ultracentrifuge for three subsequent cycles. The LDL fraction was isolated and stored under N₂, at 4°C. Before experiments, native LDL (nLDL) was dialyzed overnight at 4°C against 0.9% w/v NaCl. To obtain oxidized LDL (oxLDL) with varying degrees of oxidation, native LDL was first dialyzed against 0.15 M NaCl solution containing 1 mM NaNO₃, overnight at 4 °C. Then, nLDL was diluted to 3-5 mg/ml, and CuSO₄ was added to a final concentration of 25 µM and incubated at 37 °C. In a similar way LDL was oxidized using FeSO₄ instead of CuSO₄. Oxidation with FeSO₄ was also preceded by the dialysis step. Next, LDL was dialyzed against 5 µM FeSO₄ in PBS with additional 150 mM NaCl and 1 mM NaN₃, pH 7.2. The degree of oxidation is controlled by choosing a certain number of oxidation buffer refresh cycles. The more often FeSO₄ in buffer is refreshed each 10-12 h, the higher the degree of oxidation will be. To stop oxidation, the LDL sample is dialyzed against a buffer of 150 mM NaCl, 1 mM NaN₃, 1 mM EDTA for 4 h at 4°C. The degree of oxidation was followed by measurement of diene-formation at λ = 234 nm (Ultrospec 3000 Spectrophotometer (Pharmacia Biotech)). To stop the oxidation reaction, LDL was dialyzed against 0.15 M NaCl, 1 mM NaNO₃ and 1 mM EDTA. LDL solutions were stored at 4°C under N₂. Presence of crossbeta structure conformation in the ApoB protein portion of LDL was analyzed using a Thioflavin T fluorescence assay (see above).

### Preparation of misfolded proteins using denaturing surfaces

To prepare misfolded proteins upon exposure to surfaces composed of multimeric molecules, CpG-ODN (Coley Pharmaceutical Group, MA, USA) at 21.4 µg/ml or lipopolysaccharide (LPS, from Escherichia coli serotype O11:B4, #L2630, lot 104K4109, Sigma-Aldrich) at 600 µg/ml were mixed with 1 mg/ml of chicken egg-white lysozyme (lyophilized, Fluka, Sigma-Aldrich; catalogue number 62971), BSA, Endostatin and ovalbumin, and incubated o/n at 4°C, or for 1 h at room temperature, on a roller bank. For this purpose, lyophilized proteins were dissolved in HEPES-buffered saline (HBS, 10 mM HEPES, 4 mM KCI, 137 mM NaCl, pH 7.2) to a final concentration of 2 mg/ml, and Endostatin at 7.9 mg/ml was diluted to 2 mg/ml in HBS. Proteins were gently dissolved on a roller bank at room temperature for 10 min, at 37°C and at room temperature for 10 min. The protein solutions at 2 mg/ml were then ultracentrifuged for 1 h at 100,000*g before use, and subsequently diluted 1:1 in HBS with 42.9 µg/ml CpG-ODN or with 1200 µg/ml LPS. Formation of amyloid-like crossbeta structure was assessed by measuring enhancement of Thioflavin T fluorescence with respect to control protein solutions in which the denaturing surfaces was omitted. For this purpose, proteins were diluted to 25 µg/ml and incubated with assay buffer or with 25 µM Thioflavin T in assay buffer (see above for assay details).

Alternatively, misfolded proteins are obtained after exposure of proteins to denaturing molecules such as (negatively charged) (phospho)lipids such as phosphatidyl serine and cardiolipin, dextran sulphate (500,000 Da), alum, ellagic acid, glass or kaolin. These misfolded proteins are included in tests conducted to reveal the working mechanism of IgIV action.

### Enzyme linked immunosorbent assay for testing of IgIV binding to misfolded proteins

Binding of IgIV or of immunoglobulins in normal pooled plasma was determined using an enzyme linked immuno sorbent assay (ELISA) set-up. For this purpose 50 µl/well of potential ligands at indicated concentrations or coat buffer only for control and background measurement purposes, were coated overnight at 4°C, with motion, in 50 mM NaHCO₃ pH 9.6. Glycated albumin and Hb (BSA-AGE and Hb-AGE), control BSA and control Hb were coated at 5 µg/ml. Aβ and FP13 were coated at 25 µg/ml. The BSA and Hb controls were prepared freshly by dissolving lyophilized proteins at 1 mg/ml in PBS upon resuspending by pipetting, followed by a 30' period at the roller bank, at room temperature. The protein solutions were centrifuged for 10' at 16,000*g and diluted in coat buffer. Coat controls were performed with anti-glycated protein antibody, anti-albumin antibody, anti-Hb antibody and anti-Aβ antibody. FP13 was not recognized by a polyclonal anti-fibrinogen antibody. The alkaline phosphatase-conjugated anti-human Ig antibodies were controlled by coating the IgIV's and overlaying them the secondary antibodies. After coating the plates were washed twice with 50 mM Tris-HCl pH 7.3, 150 mM NaCl, 0.1% v/v Tween20, and blocked with 175 µl/well Blocking reagent (Roche Diagnostics, Almere, The Netherlands; catalogue number 11112589001), for 1 h at room temperature, with motion. Plates were washed twice and incubated in triplicate with indicated antibodies dilution series, plasma dilution series or controls, including binding buffer only, in the absence or presence of putative inhibitors, in binding buffer; PBS/0.1% v/v Tween20, at 50 µl/well, for 1 h at room temperature, with constant motion. After four wash cycles, secondary antibodies were added to the wells, 50 µl/well, for 45' at room temperature, with motion. RAMPO and SWARPO were used at 2000 times dilution, goat anti-human IgG antibodies were diluted 3000 times, goat anti-human IgM antibodies were diluted 1000 times. After 5 washes with wash buffer followed by two washes with PBS, binding of antibodies was assessed. For alkaline phosphatase conjugated secondary antibodies p-nitrophenyl phosphate (600 µg/ml) in DEA buffer pH 9.8 (10% v/v diethanolamine in H₂O, with 240 µM MgCl₂.6H₂O, pH adjusted with HCl) was used at 100 µl/well, for ~5'. The reaction was stopped by adding 50 µl/well of 2.4 M NaOH in H₂O. After 5' absorbance was read at 405 nm. For peroxidase-conjugated RAMPO and SWARPO, OPD (1.3 mg/ml) in 50 mM citric acid/100 mM Na₂HPO₄/0-06% v/v H₂O₂ pH 5 was used at 100 µl/well, for ~5'. The reaction was stopped by adding 50 µl/well of 2 M H₂SO₄ in H₂O. After 5' absorbance was read at 490 nm. Each experiment has been performed at least twice. To test whether amyloid-like crossbeta structure binding compounds and controls (see ref. (Bouma et al., 2003) and patent application P57716EP00) interfere with IgIV binding to crossbeta structure ligands, concentration series of the potential inhibitors were tested in the presence of a suboptimal IgIV concentration. For this purpose stock solutions used of tPA, K2P tPA, Congo red, Thioflavin S (ThS) and Thioflavin T (ThT) were 3.7 mg/ml, 1.1 mg/ml, 10 mM, 10 mM and 10 mM, respectively. The influence of tPA and K2P tPA was tested in the presence of 10 mM ε-amino caproic acid, to avoid binding of the kringle2 domain of tPA and K2P tPA to lysine- and arginine residues (tPA binding to amyloid-like structures is mediated by its finger domain, that is lacking in truncated K2P tPA; the kringle2 domain binds to exposed side chains of lysines and arginines). Binding buffer and K2P tPA serve as negative controls in these inhibition studies. Separately, similar inhibition studies were performed with immobilized Aβ or BSA-AGE, a suboptimal concentration of tPA (see ref. (Bouma et al., 2003;Kranenburg et al., 2002)) and concentration series of Congo red or ThT. Data reduction was performed as follows. Triplicates were averaged and standard deviations calculated. Background signals obtained with buffer-coated wells were subtracted (binding of primary antibody to empty wells), as well as background signals obtained with wells in which the primary antibodies were omitted (binding of secondary antibody to coated ligands).

In a separate series of experiments yeast prion peptide NH₂-GNNQQNY-COOH *(SEQ-ID 5*) was coated to the ELISA plates at a concentration of 25 µg/ml. The stock solutions of 1 mg/ml that was incubated at 4°C for 73 h was used. In control wells, 5 µg/ml Hb-AGE or coat buffer was coated. Binding of a dilutions series IgIV (I) was analyzed and compared to binding of concentration series of tPA and K2P tPA. In addition, a mixture of five monoclonal antibodies which have affinity for misfolded proteins, was also tested for binding to the immobilized ligands (see below for monoclonal details). For this purpose, a mixture comprising 336 µg/ml of each of the five antibodies was prepared in PBS, resulting in a stock solution of 1.83 mg/ml total antibody.

### Preparation of murine monoclonal anti-misfolded proteins antibodies

The immunizations were performed by the ABC-Hybridoma facility (P. van Kooten & M. Smits, Utrecht University, The Netherlands). A mouse (Balb/c) was immunized with 100 µg Aβ in 100 µl H₂O and 100 µl complete Freund's adjuvant. After three weeks, a first boost of 50 µg Aβ in H₂O-Specol (ID-DLO, Lelystad, The Netherlands) was given, followed by a second boost 30 days after the first boost. Thirty-six and 37 days after the second boost, the mouse was given two additional boosts with 50 µg Aβ in PBS (intravenously). Fourty nine weeks later, the mouse was immunized with 50 µg recombinant chicken serum amyloid A in H₂O-Specol. This antigen was a kind gift of Dr H. Toussaint (Dept. of Veterinary Medicine, University of Utrecht, The Netherlands). Four weeks later, the mouse was immunized with 50 µg Hb-AGE. Finally, 31 and 32 days later the mouse was boosted twice intravenously with 50 µg FP6 (*SEQ-ID 6*) in PBS. Three days after the final boost, the mouse was sacrificed and the spleen was used to prepare hybridomas. Fusion medium was enriched with PEG4000 (Merck, catalogue number 9727). The spleen comprised 7*10⁸ cells of which 2*10⁸ were mixed with 4*10⁷ Sp2/0 plasmacytoma cells for the fusion. After fusion selective hybridoma culture medium consisting of OptiMEM I with 10% Fetalclone1 (Hyclone), 4 µM Aminopterin and 1% Glutamax I was used. After an incubation time to allow for fusion of the spleen B-cells and the plasmacytoma cells, cells were transferred at 1 cell per well to 96-wells plates, using a Facs Vantage apparatus with Accudrop software. After approximately two weeks hybridomas were screened for putative production of anti-cross-β structure antibodies. First, 768 clones in 96-wells plates were screened for the presence of antibodies that bind to immobilized FP13 K157G amyloid and amyloid γ-globulins. For this purpose, FP13 K157G and amyloid γ-globulins were diluted together in H₂O to 5 µg ml⁻¹ of each polypeptide. Microlon high-binding ELISA plates (Greiner, Bio-One GmbH, Frickenhausen, Germany) were filled with 50 µl of this solution and air-dried overnight at 37°C. Plates were blocked with Blocking reagent (catalogue #11112589001, Roche Applied Science, Basel, Switzerland) and washed with tap water. One hundred µl of hybridoma cell culture supernatants containing 10% v/v fetal calf serum was transferred to the coated plates and incubated for 1 h at room temperature (RT) while shaking. Plates were washed with Tris-buffered saline pH 7.3 (TBS, 50 mM Tris-HCl, 150 mM NaCl) with 0.1% Tween-20 (wash buffer), and subsequently overlayed with 2000x diluted peroxidase-coupled rabbit antimouse immunoglobulins (RAMPO, #P0260, DAKO, Denmark) in PBS/0.1% Tween-20, for 30' at RT while shaking. After extensive washing, bound RAMPO was visualized with tetramethylbenzidine (TMB, #45.01.20, /#45.014.01, Biosource, Nivelles, Belgium). The reaction was stopped after 5 minutes with 1% H₂SO₄ in H₂O. Plates were read at 450 nm. Clones were included in further screening trials when signals reached at least 1.5x background levels. Again, presence of putative anti-cross-β structure antibodies was analyzed with immobilized FP13 K157G and amyloid γ-globulins. Then, 35 clones remained positive. Those clones were transferred to cell culture flasks and subjected to further analyses. For this purpose, again FP13 K157G and amyloid γ-globulins, now separately, as well as Aβ and Hb-AGE were immobilized on ELISA plates. In addition, freshly dissolved Aβ, FP13 K157G, Hb and γ-globulins were coated onto Immobilizer plates (Exiqon, Vedbæk, Denmark). These freshly dissolved controls were coated at 20, 12.5, 50 and 50 µg ml⁻¹, respectively, in PBS, for 1 h at RT while shaking. Aβ, FP13 K157G, Hb and γ-globulins stock solutions of 20, 12.5, 50 and 50 µg ml⁻¹, respectively, were first centrifuged for 30 min. at 238*10³xg to remove insoluble aggregates that might be present. Buffer was coated on Greiner (H₂O) and on Exiqon (PBS) plates as additional negative control. Greiner plates were not blocked during initial screens with 768 clones. Ten % FCS in the cell culture medium is an efficient blocker during the incubation of cell supernatant in the ELISA plates. Ten µl of PBS/1% Tween-20 was added to the wells of the Exiqon plates, before cell supernatants were added. Tween-20 at a concentration of 0.1% is an effective instant blocker for Immobilizer plates. Hundred µl of the hybridoma supernatants was transferred to the plates. Culture medium was used as negative control. Signals were calculated as multiples of the signals obtained when fresh culture medium with 10% FCS was incubated on the various immobilized antigens and controls. Signals were considered positive when exceeding 2.0x the background values obtained with fresh culture medium. Subsequent screening of 21 out of 35 clones was performed on Greiner plates, prepared as described above. The plates were now first blocked with Blocking reagent and washed. Fifty µl of each hybridoma clone supernatant was tested in duplicate for the presence of sequence independent, but structure specific antibodies, fresh culture medium was tested in fourfold as control. From the original 21 clones, six were selected for further single cell sub-cloning to obtain monoclonal hybridomas. The six clones were seeded at one cell per well of a 96-wells culture plate and cultured in medium enriched with 10% v/v FCS. The clones were all tested for binding to two coated amyloids. For each of the six clones five sub-clones were identified that bound to the two amyloids, for subsequent culturing in 25 cm² culture flasks. Isotyping of the thirty subclones using fluorescently labeled isotype-specific antibodies has been performed by the ABC-Hybridoma facility (M. Smits) according to the recommendations of the manufacturer (Luminex, Austin, TX, USA). The antibodies were purified from cell culture medium using conventional chromatographic purification technology. Samples were subjected to thiophillic chromatography using AFFI-T gel matrix (KemEnTEC, Biozym, Landgraaf, The Netherlands) in an Econo column (Biorad, Veenendaal, The Netherlands). Purified antibodies were stored at -20°C in PBS.

In order to obtain monoclonal antibodies, after a mouse was sequentially immunized with human amyloid Aβ(1-40) E22Q, recombinant chicken serum amyloid A and glycated human haemoglobin with amyloid-like properties, followed by a final boost with amyloid human fibrin peptide FP6. Hybridomas were formed and their cell culture supernatants were screened for the presence of antibodies that specifically recognize an epitope that is only recognized when cross-β structure conformation is present in any polypeptide with an amino-acid composition that is unrelated to antigens used for immunization. Out of 768 clones six clones, 2E2, 4F4, 7H1, 7H2, 7H9 and 8F2, were selected that show affinity for a broader range of amyloid-like aggregates other than the antigens used for immunization. After several rounds of selection and subcloning finally the following five monoclonal antibodies showed consistent binding to misfolded proteins with crossbeta structure conformation: 2E2B3D12, 7H2H2, 7H1C6A7, 7H9B9, 8F2G7H7. The 7H2H2 clone specifically binds only to various misfolded forms of immunoglobulins, which let us to type this clone as a 'Rheuma factor-like antibody'. A mixture of the five listed monoclonal antibodies was prepared in which the final concentrations of the individual antibodies was 1.5, 0.37, 0.4, 0.45 and 0.47 mg/ml for 2E2B3D12, 7H2 H2, 7H1C6A7, 7H9B9 and 8F2G7H7, respectively, giving an overall antibody concentration of 3.2 mg/ml. Alternatively, all antibodies were diluted in PBS to 1.83 mg/ml and combined 1:1:1:1:1 resulting in a total antibody concentration of 1.83 mg/ml with 336 µg/ml of the individual antibodies. These mixtures of murine anti-misfolded protein antibodies were used as stock solutions for further blood platelet aggregation assays (see Example).

### Platelet aggregation

The influence of IgIV on blood platelet aggregation induced by aggregates of misfolded glycated Hb with amyloid-like crossbeta structure conformation was tested with washed platelets in an aggregometric assay. Freshly drawn human aspirin free blood was mixed gently with citrate buffer to avoid coagulation. Blood was spinned for 15' at 150*g at 20°C and supernatant was collected; platelet rich plasma (PRP). Buffer with 2.5% trisodium citrate, 1.5% citric acid and 2% glucose, pH 6.5 was added to a final volume ratio of 1:10 (buffer-PRP). After spinning down the platelets upon centrifugation for 15' at 330*g at 20°C, the pellet was resuspended in HEPES-Tyrode buffer pH 6.5. Prostacyclin was added to a final concentration of 10 ng/ml, and the solution was centrifuged for 15' at 330*g at 20°C, with a soft brake. The pellet was resuspended in HEPES-Tyrode buffer pH 7.2 in a way that the final platelet number was adjusted to 200,000-250,000 platelets/µl. Platelets were kept at 37°C for at least 30', before use in the assays, to ensure that they were in the resting state. Platelets of five donors were isolated separately on three different days (2, 2, 1).

For the aggregometric assays, 270, 280 or 300 µl platelet solution was added to a glass tube and prewarmed to 37°C. A stirring magnet was added and rotation was set to 900 rpm, and the apparatus (Whole-blood aggregometer, Chrono-log, Havertown, PA, USA) was blanked. A final volume of 30, 30 or 33.3 µl was added, containing the agonist of interest and/or the premixed antagonist of interest, prediluted in HEPES-Tyrode buffer pH 7.2. Aggregation was followed in time by measuring the absorbance of the solution, that will decrease in time upon platelet aggregation. As a positive control, either 10 µg/ml collagen (Kollagenreagens Horm, NYCOMED Pharma GmbH, Linz, Austria; lot 502940), or 5 µM of synthetic thrombin receptor activating peptide TRAP (NH₂-SFLLRN-COOH, *SEQ-ID 7*) was used. Aggregation was recorded for 15' and expressed as the percentage of the transmitted light (0-100%).

### Preparation of a crossbeta structure affinity matrix for capturing proteins that bind to misfolded proteins

In order to be able to further investigate whether a subset of the Ig's in IgIV binds to misfolded proteins, we prepared an affinity matrix with linked misfolded protein. For this purpose, we coupled glycated Hb to CNBr-Sepharose (GE Healthcare-Amersham, Roosendaal, The Netherlands) according to the manufacturer's recommendations. For overnight coupling at 4°C at a rollerbank, 250 µg of HCl-washed and coupling-buffer washed, aspirated beads (dry weight) was incubated with 125 µl coupling buffer only (control beads) (100 mM NaHCO₃ pH 8.3, 500 mM NaCl) or with coupling buffer with 3.33 mg/ml Hb-AGE. After extensive washing, we determined whether Hb-AGE was coupled to the Sepharose and whether the prepared affinity matrix was indeed capable of capturing proteins that have affinity for misfolded proteins with crossbeta structure conformation. Coupling efficiency was determined by comparing the concentration of Hb-AGE starting material with the Hb-AGE supernatant after the coupling reaction. Dilution series were prepared in ADV01 protein stain (Cytoskeleton) and absorbance was read at 590 nm. Comparing absorbance signals revealed that 50% of the Hb-AGE was bound to the Sepharose, i.e. approximately 200 µg Hb-AGE at 250 µg beads (dry weight).

Previously, we established that tissue-type plasminogen activator is an enzyme with affinity for misfolded proteins with crossbeta structure conformation, including glycated proteins(Bouma et al., 2003;Kranenburg et al., 2002). To test the ability of the Hb-AGE - affinity matrix to bind tPA, twenty µl of a 1:1 suspension of Hb-AGE Sepharose or control Sepharose in HBS was incubated with 6 µM tPA (optimized concentration after testing 0-10 µM concentration series) by overnight incubation at 4°C at a rollerbank, in duplicates. After two minutes centrifugation at 8,000*g and discarding the supernatant, beads were washed five times with HBS. Bound tPA was eluted by incubating the matrix for 1 h at room temperature with 20 µl elution buffer (10 mM HEPES pH 7.4, 1140 mM NaCl, 10 mM ε-amino caproic acid, 4.5 mM CaCl₂, 0.005% Tween20). The eluate was analyzed for the tPA content and this was compared with the tPA content of the incubation mixture before and after contacting the Hb-AGE Sepharose or control Sepharose. Relative tPA concentrations were determined using a chromogenic tPA substrate S2765 (Chromogenix, Instrumentation Laboratory SpA, Milano, Italy). For this purpose, 1-5 µl of tester samples (tPA starting solutions, supernatant after contacting the Hb-AGE Sepharose, eluate after incubation of Hb-AGE Sepharose with elution buffer) was mixed with 10 µl 5 mM S2765 and 5 µl of a 10 times HBS stock solution, and adjusted with H₂O to a final volume of 50 µl. Conversion of the substrate by tPA from a colourless agent to a yellow substance was recorded in time at an absorbance 96-wells kinetic plate reader, at 37°C.

Next, 120 µl or 20 µl of the affinity Hb-AGE Sepharose matrix or 120 µl or 20 µl of the control Sepharose without coupled protein were incubated with 200 µl of the 50 mg/ml IgIV (Octagam) stock solution (4 h at room temperature). Then, the concentration of IgIV remaining in solution and the amount of bound IgIV to the matrix after extensive washing with incubation buffer, was determined. Protein concentrations were determined by measuring absorbance at 280 nm, using an IgIV standard dilution series, and by comparing absorbance at 590 nm of IgIV samples after staining with ADV01 (Cytoskeleton), with staining of an IgIV standard dilution series. IgIV bound to Hb-AGE - Sepharose or to control-Sepharose was washed six times with approximately two volumes of HBS (binding buffer). Then, bound IgIV was eluted with 200 µl HBS with 1 M NaCl and 10 mM ε-amino caproic acid (30' at room temperature, with agitation). Binding to Hb-AGE immobilized on an ELISA plate was analyzed with dilution series of untreated IgIV, IgIV after contacting Hb-AGE - Sepharose, IgIV after contacting control-Sepharose, eluted IgIV from Hb-AGE - Sepharose, eluted IgIV from control - Sepharose. For this purpose, 5 µg/ml Hb-AGE or heat-denatured BSA was coated for 1 h at room temperature, with agitation (Greiner Microlon high-binding plate). Plates were blocked with Blocking Reagent (Roche). Binding of dilutions series of the IgIV preparations was assessed as described above. The relative amount of IgIV in eluted IgIV from Hb-AGE - Sepharose and in eluted IgIV from control - Sepharose was calculated with respect to the IgIV stock. For this purpose a standard curve was prepared of a dilution series of IgIV bound to Hb-AGE or bound to heat-denatured BSA. Enrichment of the eluted IgIV from Hb-AGE - Sepharose with respect to binding to coated Hb-AGE was assessed using IgIV that was incubated with 120 µl Sepharose. Enrichment with respect to binding to heat-denatured BSA was assessed with IgIV incubated with 20 µl Sepharose.

### Immunohistochemical stain of brain sections of deceased Creutzfeldt-Jakob's disease patients with IgIV and a mixture of monoclonal anti-misfolded protein antibodies

For immunohistochemical stains of brain sections of deceased Creutzfeldt-Jakob's disease (CJD) patients with sporadic CJD or new variant CJD, paraffin sections were prepared (Dept. of Pathology, University Medical Center Utrecht, The Netherlands). The sections were applied to a standard stain procedure comprising the following steps: 1. fixed sections were blocked with block buffer, 2. incubated with IgIV or monoclonal antibodies with affinity for misfolded proteins with crossbeta structure conformation, diluted in binding buffer, 3. washed, 4. incubated with an anti-human IgG antibody and anti-murine IgG/IgM antibodies, respectively, 5. washed, 6. incubated with Powervision, 7. washed, 8. stained with DAB, and 9. enclosed and mounted, decontaminated by an acid treatment, before microscopic analysis and scoring. The procedure was performed by qualified personel in the authorized category III laboratory equipped for working with TSE-contaminated materials, located in the UMC Utrecht, The Netherlands). As control sections were incubated consecutively with tPA, a murine monoclonal anti-tPA antibody and Powervision, followed by DAB stain. As a control for the stain procedure, brain sections of a deceased Alzheimer's disease patient were also incubated with IgIV or tPA, following the same procedure as given above.

### Results & Discussion

### Example 1: IgIV (Human immunoglobulin IgG antibodies) bind to misfolded proteins comprising crossbeta structure conformation

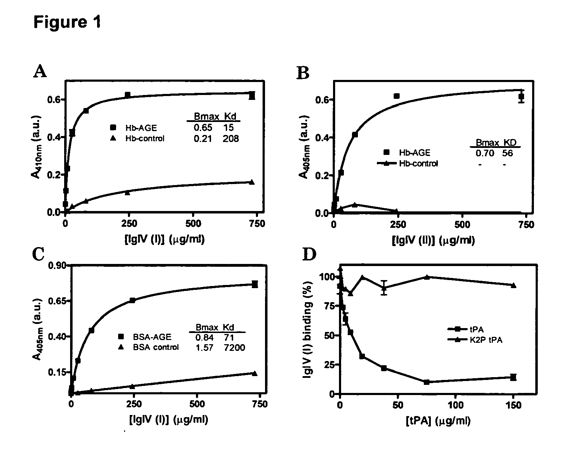

Non-enzymatic modification of proteins by carbohydrates, a process termed glycation induces protein misfolding accompanied with formation of amyloid crossbeta structure(Bouma et al., 2003). Binding of IgIV to immobilized glycated proteins Hb-AGE and BSA-AGE and non-glycated Hb and BSA was established using an ELISA set-up (Figure 1A-C). Binding of IgIV was detected using alkaline-phosphatase-labeled anti-human IgG or IgM antibodies. Both IgIV (I) and IgIV (II) bound with high affinity to glycated proteins comprising crossbeta structure, whereas they bound weakly to immobilized native albumin and native haemoglobin (Figure 1A-C). Affinity of IgIV (I) for immobilized protein was higher than of IgIV (II). Affinity of IgIV (I) for Hb-AGE was higher than for BSA-AGE. Depending on the albumin or haemoglobin preparation, a slightly varying amount of IgIV bound to these 'native' proteins, most likely due to varying amounts of molecules with a non-native conformation, exposing the binding site for IgIV antibodies with affinity for misfolded proteins.

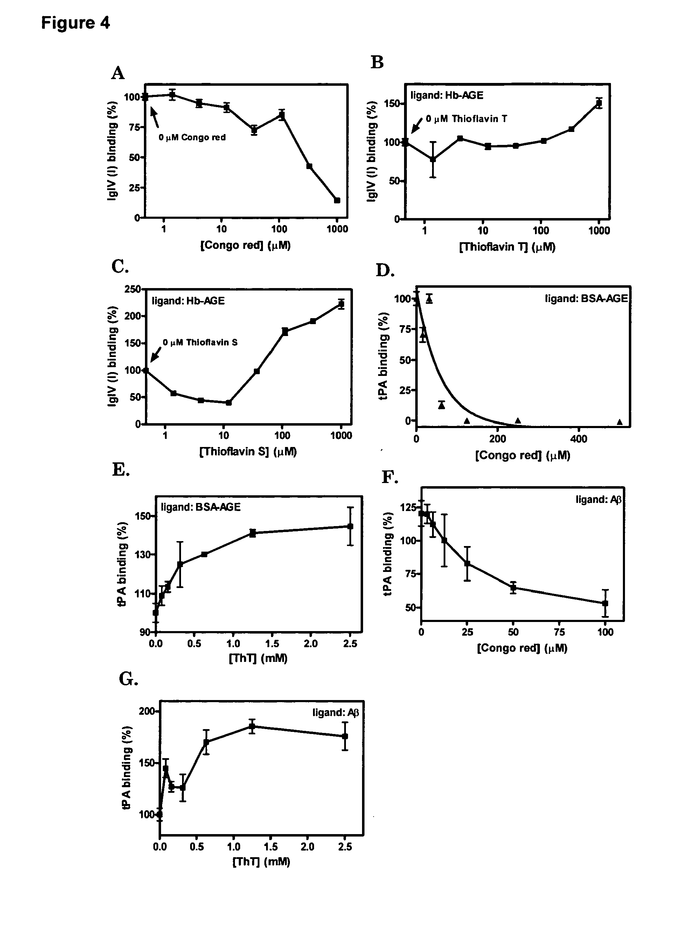

Tissue-type plasminpgen activator is a serine protease containing a module, termed the finger domain, that specifically interacts with misfolded proteins comprising crossbeta structure(Kranenburg et al., 2002;Gebbink et al., 2005). Binding of IgIV (I) at the suboptimal concentration of 15 µg/ml to coated glycated proteins is effectively diminished by a concentration series of tPA, whereas truncated K2P tPA has no influence on IgIV (I) binding (Figure 1D). It is known that tPA binds with relatively high affinity (kD of approximately 500 pM) to glycated proteins and, with somewhat lower affinity, to many other misfolded proteins with amyloid-like protein conformation comprising crossbeta structure, most likely via its fibronectin type I domain, which is lacking in K2P tPA. Similar to tPA, also the amyloid-specific dye Congo red effectively blocks the binding of 15 µg/ml IgIV (I) to coated glycated protein (Figure 4A).

To assess whether IgIV has broad-range specificity for any misfolded proteins, without limitations to the amino-acid sequence of the protein with crossbeta structure, heat-denatured MSA, ovalbumin, and glucagon were analyzed for IgIV binding, as well as oxidized ovalbumin, glucagon, haemoglobin and LDL, and the control non-oxidized or non-heat-denatured counterparts. For this purpose all proteins were immobilized on a Greiner microlon high-binding plate at 25 µg/ml concentration in 50 mM NaHCO₃ (glucagon: 12.5 µg/ml), and overlayed with a concentration series of IgIV (I), 0/1/3/9/27/81/243/729 µg/ml in PBS/0.1% v/v Tween20.

In conclusion, these results demonstrate that IgIV binds to immobilized misfolded proteins that comprise crossbeta structure conformation. To further substantiate these findings, binding to a series of misfolded proteins is assessed. For example, binding of IgIV to oxidized proteins, heat-denatured proteins, proteins denatured upon exposure to (biocompatible) surfaces, e.g. in extracorporal circulation devices, to disease related misfolded proteins (e.g. amyloid-β (Alzheimer's disease); β2-microglobulin (dialysis)), is addressed.

### Example 2: Blood platelet aggregation is induced by amyloid-like misfolded protein and is inhibited by human IgIV and murine monoclonal antibodies

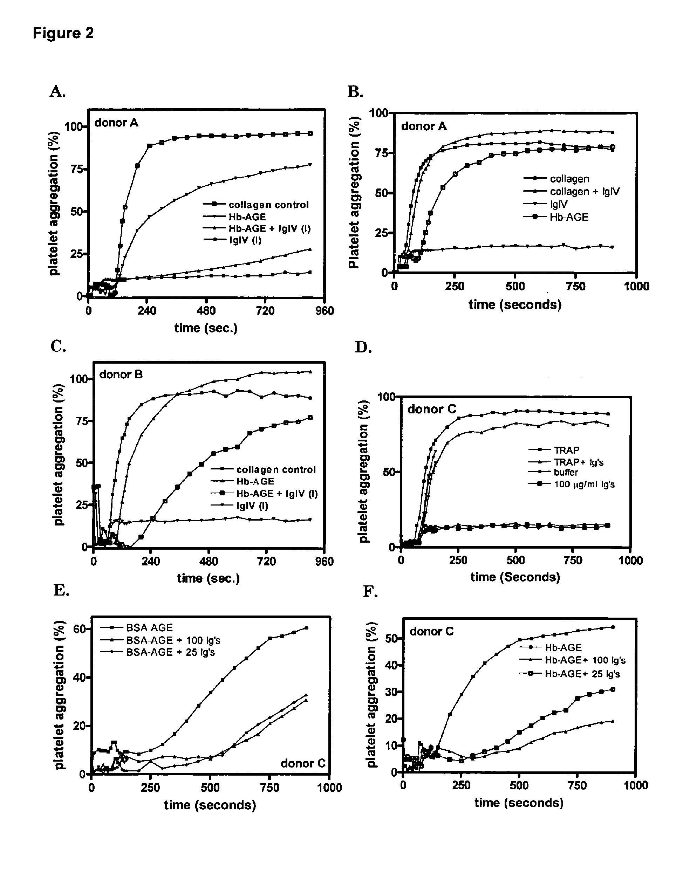

Platelets isolated from freshly drawn citrated blood of apparently healthy human volunteers readily aggregate when exposed to misfolded glycated proteins, as shown for platelets from three different individuals (donor 'A', 'B', 'C') with Hb-AGE (Figure 2). When the misfolded protein Hb-AGE or BSA-AGE is pre-incubated with IgIV (I) (Figure 2A, C) or with a mixture of five monoclonal antibodies (2E2B3D12, 7H2H2, 7H1C6A7, 7H9B9, 8F2G7H7) with affinity for misfolded proteins comprising crossbeta structure conformation (Figure 2E, F), platelet aggregation is inhibited. Induction of platelet aggregation by collagen or TRAP, is hardly influenced by the IgIV (I) or mixed monoclonal antibodies (Figure 2B, D), indicating that the monoclonal antibodies specifically inhibit the effects mediated by proteins comprising crossbeta structure.

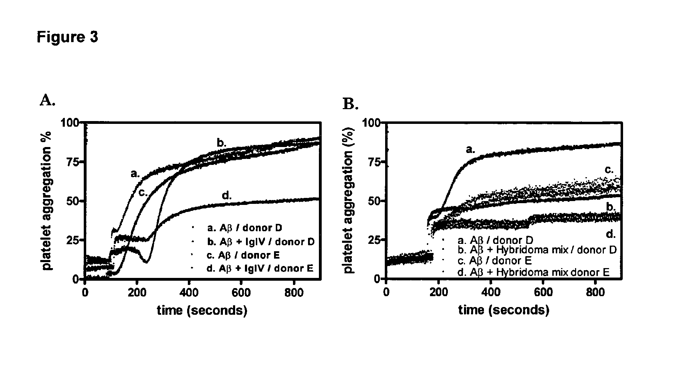

In a separate series of experiments using platelets of human donors D and E, platelet aggregation was induced by 50 µg/ml Aβ (Figure 3). The influence of 2.5 mg/ml IgIV (I) or of the monoclonal antibody mixture on amyloid-induced aggregation was addressed (Figure 3). Both IgIV (I) and the monoclonal mixture inhibit amyloid-induced platelet aggregation with platelets of two different donors (D and E). Donor D shows a higher % final aggregation than donor E upon stimulation by Aβ. For both donors, IgIV (I) delays the start of platelet aggregation by approximately 2 minutes. Platelets of donor D that are incubated with both Aβ and IgIV finally aggregate to a similar extent when compared to incubation with Aβ. With platelets of donor E, addition of IgIV to Aβ results in a stronger inhibition of platelet aggregation. Four µM TRAP was applied as a positive control. In control experiments the influence of IgIV or monoclonal antibodies on TRAP activation of platelets was analyzed by pre-incubate the TRAP stock with the mixture of monoclonal antibodies. These aggregation experiments showed that the IgIV or the monoclonal antibodies do not influence TRAP induced aggregation (not shown).

These results show that human IgIV contains antibodies that inhibit platelet aggregation induced by glycated proteins and Aβ comprising crossbeta structure. The mixture of monoclonal anti-misfolded protein antibodies exhibit a similar inhibitory activity indicative for the presence of anti-misfolded protein antibodies in the human IgIV therapeutic solution. A wide variety of misfolded proteins are now tested for their ability to induce platelet aggregation. Subsequently the influence of either human IgIV or murine anti-misfolded protein antibodies is addressed to substantiate the current findings. Alternative misfolded proteins used to induce platelet aggregation are, but are not limited to, oxidized proteins, (heat-)denatured proteins, glycated proteins, proteins exposed to denaturing surfaces or denaturing molecules, e.g. CpG-ODN, lipopolysaccharides, dextran sulphate, kaolin, glass, lipids, or amyloid peptides, e.g. FP6, amyloid-β, FP13.

### Example 3: Potentiation of binding of IgIV and tPA to misfolded protein, by Thioflavin T and Thioflavin S

Two amyloid-specific dyes, Thioflavin T and Thioflavin S, inhibit IgIV - glycated protein interaction to some extent at relatively low dye concentrations, whereas at relatively high dye concentrations both dyes seem to facilitate binding of IgIV to immobilized misfolded protein (Figure 4B, C). This is explained by the fact that binding of an amyloid-specific dye to a misfolded protein facilitates subsequent binding of a protein with affinity for binding to misfolded proteins. Thioflavin T and Thioflavin S binding stabilizes the surrounding molecules or part of molecules with crossbeta structure conformation in a relatively fixed state that represents a binding site for IgIV. At low dye concentrations, these forces are yet too weak to provoke fixation into a more uniform, IgIV-binding site exposing crossbeta structure. Now, dye binding directly competes for IgIV binding sites. At higher dye concentrations, bound dye molecules exert their stabilizing forces to the surrounding crossbeta structure in concert, thereby creating readily accessible binding sites for IgIV. Similar effects of Congo red and Thioflavin T are seen when binding of a suboptimal concentration of tPA to immobilized BSA-AGE or Aβ is considered (Figure 4D-G). The observation that binding of an amyloid-specific molecule to crossbeta structure under certain conditions facilitates binding of another molecule with specificity for misfolded proteins is used to improve the efficacy of drugs, such as antibodies, and to treat protein misfolding diseases, such as amyloidosis.

### Example 4: Misfolded protein-Sepharose affinity matrix for binding proteins with affinity for ligands with amyloid-like crossbeta structure conformation

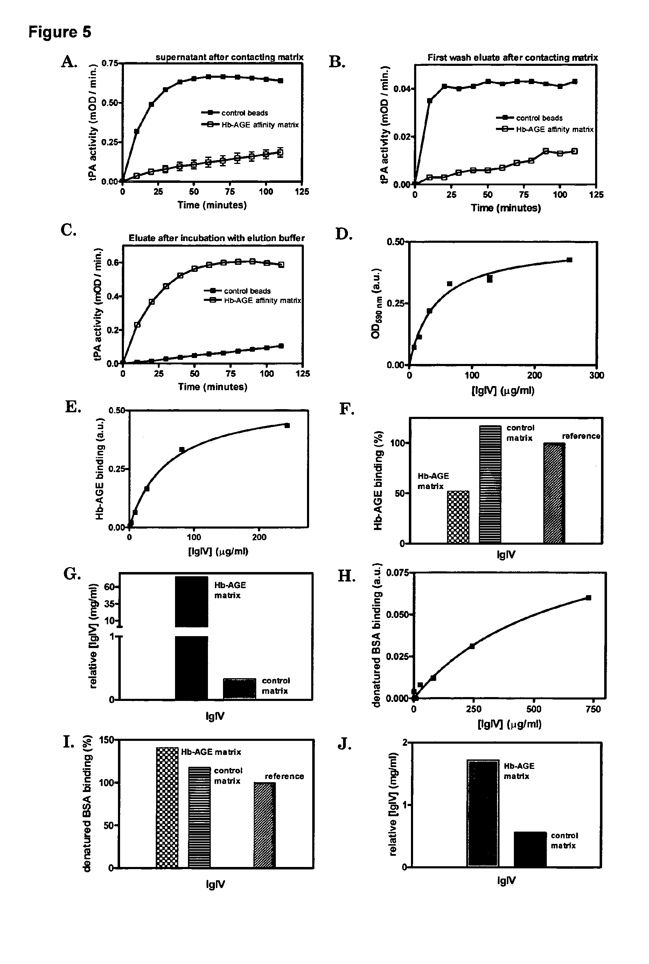

Immobilization of extensively glucose-6-phosphate glycated haemoglobin, Hb-AGE, to CNBr-Sepharose matrix resulted in an efficient affinity matrix for capturing tPA from solution (Figure 5). It is shown that tPA specifically binds to the misfolded protein affinity matrix (Figure 5A). This is further depicted by analysing the tPA content of the wash buffer after incubation of this buffer with tPA-incubated Hb-AGE misfolded protein affinity Sepharose matrix or tPA-incubated control matrix without coupled protein (Figure 5B). Hardly any tPA serine protease activity is recovered in the wash buffer after washing Hb-AGE Sepharose, and some tPA activity is seen in the wash buffer after washing tPA-incubated control beads. After incubation of tPA-incubated affinity matrix and control matrix, analysis of the recovery of tPA activity in the elution buffer shows that the Hb-AGE Sepharose is an efficient and selective affinity matrix for tPA (Figure 5C).

In a next series of experiments the Hb-AGE Sepharose affinity matrix for proteins that bind to misfolded proteins, was used to capture the fraction in IgIV that binds specifically to misfolded proteins. IgIV that specifically bound to Hb-AGE - Sepharose was tested for binding to immobilized Hb-AGE and heat-denatured BSA, in an ELISA. First a standard curve of a dilution series of the IgIV stock was prepared using protein stain ADV01 (Figure 5D). IgIV concentrations after contacting affinity matrix and after elution of bound protein from affinity matrix were determined using the IgIV standard curve. In a similar way, standard curves were prepared for the binding of dilution series of IgIV stock to immobilized Hb-AGE or heat-denatured BSA (Figure 5E, H). Relative IgIV concentrations in IgIV after contacting affinity matrix or control matrix and in the eluates was determined by calculating IgIV concentrations using the standard curves. These calculated IgIV concentrations were compared with IgIV concentrations that were determined directly using ADV01 stain. With these numbers, an enrichment factor for specific binding of IgIV to misfolded proteins is calculated. In Figure 5F, binding of 1000 times diluted IgIV stock (50 µg/ml) and IgIV contacted with Hb-AGE - Sepharose or control-Sepharose to coated Hb-AGE is shown. Hb-AGE binding is approximately 50% reduced after contacting IgIV with Hb-AGE matrix, whereas no decrease in signal is observed after contacting IgIV with control matrix. Total protein concentrations after contacting Hb-AGE matrix or control matrix were 55 and 60 mg/ml. These deviations from the maximally expected value of 50 mg/ml (starting material) result from the non-linearity of the standard curve. In the IgIV fractions eluted from Hb-AGE - Sepharose and control matrix, 120 and 7 µg/ml IgIV was present, respectively, as determined with ADV01 protein stain. When binding of the 100 times diluted eluates to immobilized Hb-AGE was assessed, the observed signals corresponded to signals obtained after binding of approximately 75 and 0.3 mg/ml IgIV stock (Figure 5G). Therefore, in conclusion, 120 µg/ml of Hb-AGE - Sepharose affinity matrix enriched IgIV binds Hb-AGE with a potency corresponding to approximately 75 mg/ml of the original IgIV stock. This corresponds to an enrichment factor of approximately 75.000/120 = 600 times. In Figure I it is depicted that contacting IgIV with Hb-AGE - Sepharose or control-matrix does not reduce the signals obtained after assessing IgIV binding to immobilized heat-denatured BSA, when compared to starting material. However, when the 120 µg/ml IgIV that was eluted from the Hb-AGE matrix, was tested for binding to heat-denatured BSA, signals corresponded to signals obtained after binding of 1.7 mg/ml starting material (original IgIV stock) (Figure 5J). This shows that contacting IgIV with misfolded protein affinity-matrix increases specificity for heat-denatured BSA with approximately 1700/120 = 14 times.

Alternative to Hb-AGE, other misfolded proteins are immobilized to a matrix in order to improve selectivity, affinity, capacity and/or stability of the affinity matrix. Alternative misfolded proteins that are immobilized are, but are not limited to, oxidized proteins, (heat-)denatured proteins, glycated proteins, proteins exposed to denaturing surfaces or denaturing molecules, e.g. CpG-ODN, lipopolysaccharides, dextran sulphate, kaolin, glass, lipids, or amyloid peptides, e.g. FP6, amyloid-β, FP13. Alternative to CNBr-Sepharose, other matrices or solid supports are applied for immobilization of the misfolded protein ligand. Preferably, the solid support is produced under good manufacturer practice (GMP) conditions, and preferably, the matrix is designated as a 'Bioprocess medium', referring to safety aspects of the matrix that are compatible with medical use with respect to humans. Other matrices/solid supports are, but are not limited to, NHS-Sepharose, Streptavidin-Sepharose, latex beads, epoxy activated solid support, e.g. crosslinked polymethacrylate, activated thiol Sepharose, Carboxylink, Profinity epoxide.

An affinity matrix is prepared using a misfolded protein that contributes to a specific disease. With this affinity matrix, those Ig's that bind the disease-associated misfolded protein with crossbeta structure conformation, are selectively isolated. After recovery of this Ig fraction, a disease-specific IgIV is obtained with higher specific beneficial outcome when used as therapy for the misfolding disease. Not only IgIV comprising solely IgG's is applied to this procedure, but every Ig fraction is tested for the presence of a beneficial subset of antibodies, e.g. antibodies of the IgM subclass. A few examples of misfolded proteins that are associated with a disease state and that are applied for the preparation of the IgIV enrichment affinity matrix are amyloid-β (Alzheimer's disease), glycated proteins (dialysis, diabetes), β2-microglobulin (dialysis), transthyretin (systemic amyloidosis). See for further examples of proteins that form misfolded crossbeta structure rich molecules and that are used for the disease-specific enrichment procedure, Tables 4 and 5.

### New constructs combining, high specificity and affinity for misfolded proteins with a clearance signal: chimera of misfolded protein binding protein with Fc domains of Ig's

Based on the findings that IgG molecules in IgIV and murine monoclonal IgGl/IgM/IgG2a antibodies bind to misfolded proteins with crossbeta structure conformation, a new molecule is designed with even higher specificity and/or affinity for misfolded proteins, combined with the ability to be prone to clearance via interaction with Fc receptors. For this purpose finger domains (F) or any other protein domain with affinity for crossbeta structure, e.g. an Ig domain of receptor for advanced glycation endproducts, a domain of (cluster II, cluster IV of) low density lipoprotein receptor related protein, a domain of the scavenger receptors A, -B-I or CD36, is fused at the DNA level or at the amino-acid level with an Fc portion of an Ig molecule. In fact, any of the proteins that has affinity for misfolded proteins provides a suitable domain to introduce specificity for crossbeta structure in the complex construct with the Fc domain (Table 4, 5). Finger domains of tPA, factor XII, hepatocyte growth factor activator and fibronectin all bind to misfolded proteins with crossbeta structure conformation, and are therefore all used for the design of chimeric constructs. Any combination of finger domains or stretches of multiple finger domains or combinations of finger domain(s) and other misfolded protein binding domains are also applied for the development of a chimeric construct with an Fc domain. The chimer gene is fused and prepared synthetically and is cloned in a suitable expression vector for expression purposes in for example yeast cells, plant cells, bacteria, eukaryotic cells, e.g. human embryonic kidney cells, baby hamster kidney cells. After purification of for example the recombinant F-Fc chimeric protein, it is applied as a therapeutic agent for any of the diseases for which IgIV has been used. Alternatively, affinity regions or synthetic molecules or any (portion of a) protein with affinity for crossbeta structure or for a protein comprising crossbeta structure are fused to for example Fc regions by any method known to a person skilled in the art for (non)-covalently coupling of protein (fragments). Moreover, non-proteinaceous molecules with affinity for crossbeta structure and/or molecules comprising crossbeta structure (Table 3) are fused to Fc regions in a similar way.

### Models to test the protective and/or beneficial effects of administering IgIV, affinity-purified enriched IgIV or chimeric structures of a misfolded protein binding protein or molecule and an Fc domain

To test a beneficial effect of IgIV, or an enriched IgIV fraction after affinity purification with a matrix with coupled misfolded protein, (humanized) anti-misfolded protein antibodies, or a chimeric structure of for example a finger domain and an Fc domain of an IgG molecule, several *in vitro* cell-based models for disease states, as well as *in vivo* animal or human models are applied to determine whether such modalities have a more pronounced beneficial effect than administering total IgIV or than current standard therapy.

### In vitro murine dendritic cell assay

(Auto)immunity is dependent on the presentation of (auto)antigens by antigen presenting cells, such as dendritic cells. Cultured murine dendritic cells (DC's) are thus applied as a model for (auto)immunogenicity. For this purpose, DC's are isolated from the hind legs of for example 8-12 weeks old Black-6 mice. Bones are isolated and rinsed in 70% ethanol, rinsed in RPMI-1640 medium with 25 mM HEPES, with 10% fetal calf serum, penicillin and Streptomycin. Then the bone is flushed with this buffer, in both directions. Eluates are cleared from erythrocytes by adding erythrocyte specific lysis buffer (obtained from the local UMC Utrecht Pharmacy Dept., catalogue number 97932329). Eluates are analyzed for viable cells by culturing them in cell culture plates. At this stage, the medium is enriched with 10 ng/ml GM-CSF. DC's growth in suspension or on a layer of macrophage cells. Using a FACS and specific antibodies, it is determined whether DC's are present and activated. Preferably the levels of so-called co-stimulatory molecules, such as B7.1, B7.2, MHC class II, CD40, CD80, CD86 are determined on preferably CD11c positive cells. Alternatively, activation of NF-κB and/or expression of cytokines is used as indicators of activation of cells involved in immunogenicity, such as APC and DC. Preferably, the following cytokines are quantified: TNFα, IL-1, IL-2, IL-6, and/or IFNγ. Preferably, the cytokine levels are quantified by ELISA. Alternatively, the mRNA levels are quantified. For a person skilled in the art it is evident that function of APC and DC are tested as well.

Alternatively, a stable DC line, cultured dendritic cells obtained from monocytes collected from human blood or other antigen presenting cells are used to test beneficial effects of depletion or neutralisation of misfolded proteins with crossbeta structure (Citterio et al., 1999).

Further experiments that are performed with DC's are exposure of the cells to lipopolysaccharide (LPS), followed by reading out levels of the above mentioned activation markers. The effect of pre- and/or co-incubations of LPS with (enriched) IgIV and/or other affinity regions before or during exposure of the LPS to DC's, is also tested. These experiments are seen as a model for bacterial infection and sepsis in humans.

### In vitro human umbilical vein endothelial cell assay

Glycated proteins comprising crossbeta structure induce inflammatory response, believed to contribute to pathogenesis of certain diseases including diabetic nephropathy. In general, misfolded proteins induce cellular dysfunction with enhanced expression or activation of inflammatory signals. The effect of misfolded proteins on endothelial cell (dys)function is for example measured by determining the levels of reactive oxygen species in response to misfolded proteins. Human umbilical vein endothelial cells that are isolated and cultured, according to standard protocols, are used or other endothelial cells such as bEnd.3 endothelial cells. The levels of reactive oxygen species (ROS) levels are monitored using fluorescent probes, such as CM-H₂DCF-DA. Alternatively cell viability is monitored by MTT-assay. The cultured primary cells provide the opportunity to perform in vitro cell assays that are accepted in research community as model systems for certain disease states. Again, the ability of IgIV, isolated fractions thereof, a functional equivalent or our anti-crossbeta antibodies are applied in these systems.

### In vivo murine model of disseminated intravascular coagulation

Crossbeta structure induces disseminated intravascular coagulation (DIC). As a model for DIC, in female C57B1/6 mice the generalized Shwartzman's reaction is elicited. For this purpose, mice are injected with 5 µg lipopolysaccharide (LPS) in the footpad at day = 0 and with 300 µg LPS intravenously at t = 24 h. In time, survival is monitored, together with several plasma levels of proteins, e.g. cytokines.

### In vivo mouse/rat experimental autoimmune encephalomyelitis model

To test whether anti-misfolded protein antibodies provide a beneficial effect during a multiple sclerosis (MS) relapse, an in vivo mouse model for MS, the experimental autoimmune (or allergic) encephalomyelitis (EAE) model is used. For this purpose, myelin basic protein (MBP) or myelin oligodendrocyte glycoprotein peptide 35-55 (MOG35-55) is emulsified in incomplete Freund's adjuvant (IFA) with mycobacterium. The presence of misfolded proteins is determined using Thioflavin T and Congo red fluorescence assays, as well as tPA binding and activation assays. Binding of IgIV or an enriched fraction of IgIV after affinity purification, to the emulsified MBP or MOG35-55 is assessed. To induce EAE in mice or rats, the emulsified MBP or MOG35-55 is injected in for example the hind footpad. In mice a subcutaneously injected amount of MOG35-55 is preferably accompanied with an intraperitoneal injection of Bordetella pertussis toxin, which is repeated after 48h. For example, Lewis female rats are used, or female Balb/c mice. Measures for clinical disease are for example scored as follows: 0, normal; 1, limp tail; 2, impaired righting reflex; 3, paresis of hind limbs; 4, complete paralysis of hind limbs; 5, death. The effect of any (chimeric) antibody preparation is analyzed by administering the drug at one or more time points after inducing EAE. One of the preparations that is tested is IgIV that is affinity purified on an affinity matrix with immobilized denatured/misfolded MBP or MOG35-55, depending on which of the two proteins is used for inducing the disease.

### In vivo collagen induced arthritis model

In the in vivo collagen induced arthritis model, rats are injected intradermally at the base of the tail and on the back above each leg with type II (bovine) collagen, dissolved in acetic acid and emulsified in IFA. The rats are daily examined for disease signs by monitoring swelling and erythema. One of the preparations that are tested is IgIV that is affinity purified on an affinity matrix with immobilized denatured/misfolded collagen in IFA.

### In vivo mouse sepsis model

Sepsis is mediated by crossbeta structure. One of the in vivo mouse sepsis models that is applied to test effects of IgIV, monoclonal antibodies or related drugs, is the 'cecal ligation and puncture' model. For this model, female Balb/c mice are anesthetized before an abdominal incision is made to bring the cecum outside the abdomen. After puncturing the cecum an amount of luminal contents is transferred outside through the punctures, before the cecum is returned in the adomen and the mouse is closed. Infection progression is monitored by measuring the body temperature and by scoring the mobility of mice. One considers mice lethally infected when they are hypothermic (T < 33°C) and when mice are unable to right themselves. Effects of administering antibodies with affinity for misfolded proteins with crossbeta structure conformation after the puncture of the cecum are assessed by monitoring a group of untreated mice and a group of mice that received an (enriched) IgIV, monoclonal antibodies or a chimeric construct.

### In vivo rat sepsis model

As an alternative for the mouse sepsis model, the rat sepsis model is used. For example, endotoxic shock is induced in Fischer rats of approximately 150 gr. by intravenous injection of 15mg/kg *Escherichia coli* endotoxin. As a measure for disease progression, in ELISA's levels of tissue necrosis factor and interleukin-1 in blood are monitored. Effects of treatment with any preparation of IgIV or antibody or chimeric construct with affinity for unfolded protein are assessed this way.

### In vivo mouse reactivated Streptococcus cell wall-induced arthritis model

In an in vivo mouse model of reactivated Streptococcus cell wall-induced arthritis, C57BL/6 mice are induced by an intraarticular injection in the knee joints of cell walls of Streptococcus pyogenes T12 organisms. The injection is repeated five times with 1-week intervals. The disease progression is followed for example for about 40 days by measuring swelling of injected knee joints. After killing the mice, severity of the arthritis is scored macroscopically after removing the skin from the knee joints. Effects of administering anti-crossbeta structure antibodies or chimera are compared with controls that received no therapeutic and with control mice that were injected with buffer.

### In vivo mouse experimental rheumatoid arthritis model

In an in vivo mouse model for experimental collagen induced rheumatoid arthritis, for example male mice of the DBA/1 strain and/or male mice of the C57BL/6 strain are challenged with native bovine collagen type II. Arthritis is induced by injecting collagen emulsified in complete Freund's adjuvant with Mycobacterium tuberculosis, subcutaneously at the base of the tail. Mice are boosted at day 21 with collagen emulsified in IFA. Mice are monitored for evidence of arthritis and the severity of the disease is scored, using a standard scoring procedure. The effect of an antibody-based therapy is assessed by comparing control mice with arthritis and control mice that were injected twice with buffer only, with IgIV/monoclonal antibody/chimeric construct treated mice after induction of arthritis.

### In vivo human inflammation/immunogenicity model: administration of glycated protein +/- IgIV

Glycated proteins comprising crossbeta structure induce an inflammatory response, contributing to pathogenesis of certain diseases including diabetic nephropathy. In general, misfolded proteins induce cellular dysfunction with enhanced expression or activation of inflammatory signals. The inflammatory effects of misfolded proteins and anti-crossbeta structure reagents such as IgIV, fractions thereof, or functional equivalents inflammation are studied in mice and humans. Proteins comprising crossbeta structure are infused by intravenous administration. At different time intervals the effect on the level of acute phase proteins, such as C-reactive protein, Serum Amyloid A (SAA), Serum amyloid P-component (SAP) or complement factor 3 (C3) is measured. Alternatively the effect on other markers of inflammation, such as IL-6, IL-8, D-dimer or prothombin F1+2 levels is determined. Finally the levels of (auto)antibody formation are determined by ELISA.

### Whole blood assay for determination of the inflammatory or immunogenic nature of compounds

One way of assessing whether activation of cells of the immune system by proteins with crossbeta structure conformation is blocked using crossbeta structure binding compounds, e.g. IgIV, monoclonal anti-crossbeta structure antibodies, chimeric constructs, is by use of a 'whole blood' assay. For this purpose, at day 1 freshly drawn human EDTA-blood is added in a 1:1 ratio to RPMI-1640 medium (HEPES buffered, with L-glutamine, Gibco, Invitrogen, Breda, The Netherlands), that is prewarmed at 37°C. Subsequently, proteins comprising crossbeta structure conformation, with or without crossbeta structure binding compounds, are added. Preferably, a positive control is included, preferably LPS. An inhibitor that is used for LPS is Polymyxin B, at 5 µg ml⁻¹ final concentration. Standard crossbeta structure conformation rich polypeptides that are tested are Aβ, amyloid γ-globulins, glycated proteins, FP13, heat-denatured OVA, heat-denatured BSA, heat-denatured MSA, heat-denatured lysozyme, and β2gpi exposed to cardiolipin. Negative controls are native γ-globulins, native albumin, native Hb, freshly dissolved Aβ or FP13, native OVA, other native proteins. As a control, all protein samples are tested in the absence or presence of 5 µg ml⁻¹ Polymyxin B to exclude effects seen due to putative endotoxin contaminations. In addition, native proteins alone or pre-exposed to denaturing adjuvants, e.g. LPS, and CpG-ODN, or other denaturing compounds or denaturing conditions (e.g. Cu²⁺-oxidation), are tested for immunogenic activity. All aforementioned tester compounds are tested in the absence and presence of a concentration series of a potential inhibitor of the inflammatory or immunogenic response, e.g. IgIV, monoclonal anti-crossbeta antibodies. The final volume of activators, controls and potential inhibitors added to the blood-medium mixture is approximately 1/200 of the total volume. Higher concentrations of activators and putative inhibitors are achieved by using concentrated RPMI-1640 medium for predilution steps (RPMI-1640 Medium powder, Gibco, Invitrogen; catalogue number 51800-035). The blood and the medium are mixed carefully and incubated overnight in a CO₂ incubator with lids that allow for the entrance of CO₂. At day 2 medium is collected after 10' spinning at 1,000*g, at room temperature. The cell pellet is stored frozen. The medium is again spinned for 20' at 2,000*g, at room temperature. Supernatant is analyzed using ELISAs for concentrations of markers of an immune response, e.g. tissue necrosis factor-α (TNFα), cytokines, chemokines. For example, TNFα levels after exposure of whole blood to tester compounds is assessed by using the commercially available TNF-alpha/TNFSF1A ELISA (R&D Systems, Minneapolis, MN, USA; Human TNF-alpha Quantikine HS PharmPak). When positive and negative controls are established as well as a reliable titration curve, any solution is tested for the crossbeta structure load with respect to concentrations of markers for immunogenicity. Furthermore, putative inhibitors of the immune response are tested. For example, IgIV and monoclonal anti-crossbeta antibodies prevent an immune response upon addition to misfolded protein solutions.

### Phagocytosis of cross-β structure comprising moieties.

The uptake of cross-β structure comprising proteins, polypeptides and/or peptides as well as cells or cellular particles, and the effect of IgIV or a functional equivalent thereof are studied in vitro using cultured cells, preferably monocytes, dendritic cells, or macrophages or similar cells, for example U937 or THP-1 cells. Preferably, cross-β structure comprising molecules are labelled, preferably with 125I or a fluorescent label, preferably FITC, covalently attached to the molecule by a linker molecule, preferably ULS (universal Linkage system) or similar coupling method. Cells are preferably labelled with mepacrin or other fluorescent labels, such as rhodamine. Phagocytic cells are incubated in the presence of labelled cross-β structure comprising molecules or cells in the presence or absence of a cross-β structure binding compound, such as IgIV or functional equivalent thereof. After incubation, preferably during several hours, the uptake of labelled molecules or cells is measured preferably using a scintillation counter (for 125I) or by FACS-analysis (with fluorescent probes) or immunofluorescent microscopy. The uptake of cells is also counted under a light microscope with visual staining of the cells.

### Tables:

| **Table 1: reported side effects related to administering IgIV to patients‡** | | | |
|---|---|---|---|
| Venous thrombosis | arterial thrombosis | Headache | chills |
| nausea | fever | Cramping | tachycardia |
| aseptic meningitis | (acute) renal failure | Anaphylaxis | thromboembolic events |
| Pseudohyponatremia | back pain | passagere headache | seizures |
| hypotension | haemolytic anaemia | haemolytic hemolysis | nephro-toxicity |
| intolerance (anti-IgA antibodies when IgA deficient | Pseudohyponatraemia in newborn | reduced immune response against some living virus vaccines (mumps, measles, varicella/rubella vaccine) | Acquired von Willebrand's syndrome in association with a lupus-like anticoagulant |
| exanthema | eczema | pure red cell aplasia | fatigue |
| cerebrovascular accidents | Hyperviscosity in newborn | Acute myocardial ischemia | transient ischaemic attacks |
| Transient neutropenia | Acute renal transplant injury | Acute myocardial infarction | Hemolytic uremic syndrome |
| pain at injection site | | | |

| | | | |
|---|---|---|---|
| ‡ Data is retrieved from literature references obtained by Pubmed data mining, and from Octagam and Gammagard datasheets. | | | |

| **Table 2: Sequence identities of synthetic peptides** | | |
|---|---|---|
| peptide | Sequence identity | Amino-acid sequence |
| FP13 K157G | SEQ-ID 1 | KRLEVDIDIGIRS |
| Aβ(1-40) | SEQ-ID 2 | DAEFRHDSGYEVHHQKLVFFAEDVGSNKGAIIGLMVGGVV |
| Aβ(1-40) E22Q | SEQ-ID 3 | DAEFRHDSGYEVHHQKLVFFAQDVGSNKGAIIGLMVGGVV |
| FP10 | SEQ-ID 4 | KRLEVDIDIK |
| Yeast prion peptide | SEQ-ID 5 | GNNQQNY |
| FP6 | SEQ-ID 6 | IDIKIR |
| TRAP | SEQ-ID 7 | SFLLRN |
| PPACK | SEQ-ID 8 | FPR-chloromethylketone |

| **Table 3: cross-β structure conformation binding compounds** | | |
|---|---|---|
| Congo red | Chrysamine G | Thioflavin T |
| 2-(4'-(methylamino)phenyl)-6-methylbenzothiaziole | Any other amyloid-binding dye/chemical | Glycosaminoglycans |
| Thioflavin S | Styryl dyes | BTA-1 |
| Poly(thiophene acetic acid) | conjugated polyeclectrolyte PTAA-Li | |

| **Table 4: proteins that bind to and/or interact with cross-β structure and/or with proteins comprising cross-β structure** | | |
|---|---|---|
| Tissue-type plasminogen activator | Finger domain(s) of tPA, factor XII, fibronectin, HGFA | Apolipoprotein E |
| Factor XII | Plasmin(ogen) | Matrix metalloprotease-1 |
| Fibronectin | 75kD-neurotrophin receptor (p75NTR) | Matrix metalloprotease-2 |
| Hepatocyte growth factor activator | α2-macroglobulin | Matrix metalloprotease-3 |
| Serum amyloid P component | High molecular weight kininogen | Monoclonal antibody 2C11 (F8A6)‡ |
| C1q | Cathepsin K | Monoclonal antibody 4A6(A7)^{‡} |
| CD36 | Matrix metalloprotease 9 | Monoclonal antibody 2E2(B3)^{‡} |
| Receptor for advanced glycation endproducts | Haem oxygenase-1 | Monoclonal antibody 7H1 (C6)^{‡} |
| Scavenger receptor-A | low-density lipoprotein receptor -related protein (LRP) | Monoclonal antibody 7H2(H2)^{‡} |
| Scavenger receptor-B | DnaK | Monoclonal antibody 7H9(B9) ^{‡} |
| ER chaperone ERp57 | GroEL | Monoclonal antibody 8F2(G7)^{‡} Monoclonal antibody 4F4 |
| calreticulin | VEGF165 | |
| Monoclonal conformational antibody WO1 (ref. (O'Nuallain and Wetzel, 2002)) | Monoclonal conformational antibody WO2 (ref. (O'Nuallain and Wetzel, 2002)) | Amyloid oligomer specific antibody (ref. (Kayed et al., 2003)) |
| formyl peptide receptor-like 1 | α(6)β(1)-integrin | CD47 |
| Rabbit anti-albumin-AGE antibody, Aβ-purified^{a)} | CD40 | apo A-I belonging to small high-density lipoproteins |
| apoJ/clusterin | 10 times molar excess PPACK, 10 mM εACA, (100 pM - 500 nM) tPA²⁾ | CD40-ligand |
| macrophage scavenger receptor CD163 | broad spectrum (human) immunoglobulin G (IgG) antibodies (IgIV, IVIg) | |

| | | |
|---|---|---|
| ‡ Monoclonal antibodies developed in collaboration with the ABC-Hybridoma Facility, Utrecht University, Utrecht, The Netherlands. a) Antigen albumin-AGE and ligand Aβ were send in to Davids Biotechnologie (Regensburg, Germany); a rabbit was immunized with albumin-AGE, antibodies against a structural epitope were affinity purified using a column with immobilized Aβ. 2) PPACK is Phe-Pro-Arg-chloromethylketone (SEQ-ID 8), εACA is ε-amino caproic acid, tPA is tissue-type plasminogen activator | | |

| **Table 5: Proteins likely to be involved in the *'Cross-β structure pathway'*** | | |
|---|---|---|
| Monoclonal antibody 4B5 | Heat shock protein 27 | Heat shock protein 40 |
| Monoclonal antibody 3H7^{‡} | Nod2 (= CARD15) | Heat shock protein 70 |
| FEEL-1 | Pentraxin-3 | HDT1 |
| LOX-1 | Serum amyloid A proteins | GroES |
| MD2 | Stabilin-1 | Heat shock protein 90 |
| FEEL-2 | Stabilin-2 | CD36 and LIMPII analogous-I (CLA-1) |
| Low Density Lipoprotein | LPS binding protein | CD14 |
| C reactive protein | CD45 | orosomucoid |
| integrins | alpha-1 antitrypsin | apo A-IV-Transthyretin complex |
| albumin | Alpha-1 acid glycoprotein | β2-glycoprotein I |
| Lysozyme | Lactoferrin | megalin |
| Tamm-Horsfall protein | Apolipoprotein E3 | Apolipoprotein E4 |
| Toll-like receptors | Complement receptor CD11b/CD18 (Mac-1, CR3) | CD11d/CD18 (subunit aD) |
| CD11b2 | CD11a/CD18 (LFA-1, subunit aL) | CD11c/CD18 (CR4, subunit aX) |
| Von Willebrand factor | Myosin | Agrin |
| Perlecan | Hsp60 | b2 integrin subunit |
| proteins that act in the unfolded protein response (UPR) pathway of the endoplasmic reticulum (ER) of prokaryotic and eukaryotic cells | proteins that act in the endoplasmic reticulum stress response (ESR) pathway of prokaryotic and eukaryotic cells | Macrophage receptor with collagenous structure (MARCO) |
| 20S | HSP16 family members | HSC73 |
| HSC70 | translocation channel protein Sec61p | 26S proteasome |
| 19S cap of the proteasome (PA700) | UDP-glucose:glycoprotein glucosyl transferase (UGGT) | carboxy-terminus of HSP70-interacting protein (CHIP) |
| Pattern Recognition Receptors | Derlin-1 | calnexin |
| Bcl-2 asociated athanogene (Bag-1) | GRP94 | Endoplasmic reticulum p72 |
| (broad spectrum) (human) immunoglobulin M (IgM) antibodies | | |

| | | |
|---|---|---|
| ‡ Monoclonal antibodies developed in collaboration with the ABC-Hybridoma Facility, Utrecht University, Utrecht, The Netherlands. | | |

### Brief description of the drawings

### Figure 1. Human IgIV binds specifically to misfolded glycated proteins.

In ELISA set-ups the binding of human IgIV for therapeutical usage, obtained from two manufacturers, I and II, was assessed with immobilized glycated proteins. **A.** Binding of IgIV from manufacturer I (IgIV (I)) to coated glycated human haemoglobin (Hb-AGE), freshly dissolved Hb and aggregated amyloid-β peptide (Aβ) was tested. **B.** Binding of IgIV from manufacturer II (IgIV (II)) to coated Hb-AGE, freshly dissolved Hb and aggregated Aβ was tested. **C.** Binding of IgIV (I) to coated glycated albumin (BSA-AGE), freshly dissolved control albumin and FP13 K157G amyloid was analyzed. **D.** The influence of tPA and K2P tPA on the binding of 15 µg/ml IgIV (I) to coated Hb-AGE was addressed by adding concentration series of tPA or K2P tPA to the IgIV (I) incubation mixture. Ten mM of εACA was added to the mixture to avoid binding of tPA to exposed lysine or arginine side chains.

### Figure 2: Platelet aggregation induced by misfolded glycated proteins with amyloid-like conformation is inhibited by IgIV and a mixture of monoclonal antibodies.

Platelet aggregation after introduction of collagen or TRAP (positive controls), buffer (negative control) or misfolded amyloid-like glycated albumin or haemoglobin was followed in an aggregometer using isolated platelets from freshly drawn citrated plasma in HEPES-Tyrode buffer. The proposed inhibitory properties of human IgIV and murine monoclonal antibodies raised against four different amyloid structures, on platelet aggregation was assessed. **A.** IgIV purchased from manufacturer I effectively inhibits the glycated haemoglobin-induced aggregation of platelets of human donor 'A'. IgIV (I) itself has no effect on platelets, that is to say, no aggregation is induced by adding IgIV (I) to platelets. IgIV (I) concentration used was 4.7 mg/ml, the Hb-AGE concentration was 18 µg/ml, collagen was used at a concentration of 10 µg/ml. **B.** Influence of 10 µg/ml collagen, 18 µg/ml Hb-AGE, 4.7 mg/ml IgIV (I) and 18 µg/ml Hb-AGE that was preincubated with 4.7 mg/ml IgIV (I) on aggregation of platelets of donor A was determined. **C.** Similar to the experiment performed with platelets of donor A **(A.),** platelet aggregation with platelets of donor 'B' was followed in time. Now, 10 µg/ml collagen, 90 µg/ml Hb-AGE, 4.7 mg/ml IgIV (I) and 90 µg/ml Hb-AGE preincubated with 4.7 mg/ml IgIV (I) was used. **D.** In a control experiment with platelets of donor 'C' 5 µM TRAP was used as a positive control. The influence of 100 µg/ml of a mixture of five monoclonal antibodies with affinity for misfolded proteins with crossbeta structure conformation was determined with TRAP as activator of aggregation, or with HEPES-Tyrode buffer control. **E., F.** Platelet aggregation (donor C) was induced by 25 µg/ml glycated bovine serum albumin (BSA-AGE, **E.)** or glycated human haemoglobin (Hb-AGE, **F.).** Inhibition of this aggregation by 25 or 100 µg/ml mixture of five monoclonal antibodies with affinity for misfolded proteins with crossbeta structure conformation was determined.

### Figure 3: Blood platelet aggregation is induced by amyloid-β, and inhibited by IgIV or monoclonal antibodies.

**A., B.** Induction of platelet aggregation by 50 µg/ml amyloid-β is inhibited when 2.5 mg/ml IgIV (I) is pre-incubated with amyloid-β **(A.),** or when 160 µg/ml mixture of five monoclonal antibodies that bind to misfolded proteins **(B.)** is preincubated with amyloid-β. Platelets of two donors D and E are analyzed separately.

### Figure 4. Amyloid-specific small compounds influence binding of IgIV or tPA to immobilized misfolded proteins differently.

In ELISA set-ups binding of IgIV or tPA, a multiligand binding protein with affinity for misfolded proteins that comprise the crossbeta structure tertiary/quarternary fold, was analyzed under influence of concentrations series of amyloid-specific dyes Congo red and Thioflavin T. **A-C.** The influence of amyloid-specific dyes Congo red **(A.),** Thioflavin T **(B.)** and Thioflavin S **(C.)** on binding of 15 µg/ml IgIV (I) to immobilized Hb-AGE was addressed by preincubating the IgIV (I) with concentration series of the three dyes before adding the solutions to ELISA plates. **D., F.** Influence of Congo red on binding of a suboptimal concentration of tPA to coated BSA-AGE **(D.)** or Aβ **(F.). E., G.** Influence of Thioflavin T on binding of a suboptimal concentration of tPA to coated BSA-AGE **(E.)** or Aβ **(G.).**

### Figure 5. An affinity matrix with the ability to bind proteins that bind to misfolded proteins with crossbeta structure conformation.

Glycated and misfolded human haemoglobin was linked to CNBr-Sepharose and the ability to bind proteins with affinity for misfolded proteins that comprise the crossbeta structure fold was determined by analyzing tPA binding. Next, the affinity matrix was applied to isolate a subset of immunoglobulin molecules from IgIV-I comprising affinity regions for cross-β structure and/or proteins comprising cross-β structure. **A.** Hb-AGE Sepharose and empty control beads were incubated with 6 µM tPA solution and the supernatant was subsequently analyzed for the presence of tPA activity by adding tPA chromogenic substrate S2765. **B.** After incubation with tPA the Hb-AGE Sepharose and the control beads were washed several times with wash buffer. The presence of tPA in the first wash eluate was again analyzed by following tPA substrate S2765 conversion at 37°C in time. **C.** After extensive washing bound tPA was eluted from empty control Sepharose beads and Hb-AGE Sepharose with high salt. Ten times diluted eluate was analyzed for the presence of tPA by adding S2765. **D.** Standard curve of the absorbance at 590 nm of diluted IgIV stock (Octagam), stained with ADV01. **E.** Standard curve of binding of a dilution series of IgIV stock (Octagam) to Hb-AGE, as determined with ELISA. **F.** Binding to immobilized Hb-AGE of 1000 times diluted IgIV stock, IgIV after contacting with Hb-AGE - Sepharose and IgIV after contacting with control matrix, as assessed with an ELISA. **G.** Binding to immobilized Hb-AGE of IgIV eluted from Hb-AGE - Sepharose and IgIV eluted from control matrix, as assessed with an ELISA. Signals are given as relative numbers, as calculated from the IgIV stock binding curve (See Figure E). **H.** Standard curve of binding of a dilution series of IgIV stock (Octagam) to heat-denatured BSA, as determined with ELISA. **I.** Binding to immobilized heat-denatured BSA of 1000 times diluted IgIV stock, IgIV after contacting with Hb-AGE - Sepharose and IgIV after contacting with control matrix, as assessed with an ELISA. **J.** Binding to immobilized heat-denatured BSA of IgIV eluted from Hb-AGE - Sepharose and IgIV eluted from control matrix, as assessed with an ELISA. Signals are given as relative numbers, as calculated from the IgIV stock binding curve (See Figure H).

### References

Bouma,B. et al. Glycation induces formation of amyloid cross-β structure in albumin. J. Biol. Chem. 278, 41810-41819 (2003)
Citterio,S. et al. Dendritic cells as natural adjuvants. Methods 19, 142-147 (1999)
Gebbink,M.F., Claessen,D., Bouma,B., Dijkhuizen,L. & Wosten,H.A. 2005. Amyloids--a functional coat for microorganisms. Nat. Rev. Microbiol. 3, 333-341
Horbach,D.A., van Oort,E., Donders,R.C., Derksen,R.H. & de Groot,P.G. Lupus anticoagulant is the strongest risk factor for both venous and arterial thrombosis in patients with systemic lupus erythematosus. Comparison between different assays for the detection of antiphospholipid antibodies. Thromb. Haemost. 76, 916-924 (1996)
Kayed,R., E.Head, J.L.Thompson, T.M.McIntire, S.C.Milton, C.W.Cotman, and C.G.Glabe. 2003. Common structure of soluble amyloid oligomers implies common mechanism of pathogenesis. Science 300:486-489 Common structure of soluble amyloid oligomers implies common mechanism of pathogenesis. Science 300: 486-489
Kranenburg,O., B.Bouma, L.M.Kroon-Batenburg, A.Reijerkerk, Y.P.Wu, E.E.Voest, and M.F.Gebbink. 2002. Tissue-type plasminogen activator is a multiligand cross-beta structure receptor. Curr. Biol. 12:1833-1839
O'Nuallain,B. & Wetzel,R. Conformational Abs recognizing a generic amyloid fibril epitope. Proc. Natl. Acad. Sci. U. S. A 99, 1485-1490 (2002)

## Claims

1. A method for selecting from a collection of IgIV molecules, at least one IgIV molecule comprising an affinity region that is capable of interacting with an epitope of a cross-β structure and/or with an epitope of a protein comprising a cross-β structure, said method comprising contacting a collection of IgIV molecules with a cross-β structure and/or a protein comprising a cross-β structure and collecting at least one IgIV molecule comprising an affinity region interacting with said epitope.

2. A method according to claim 1, wherein said epitope is at least part of a cross-β structure of a protein.

3. A method according to claim 1, wherein said epitope is exposed on said protein comprising a cross-β structure.

4. A method according to any one of claims 1-3, wherein said cross-β structure and/or protein comprising a cross-β structure is bound to a solid support.

5. A collection of IgIV molecules, enriched in IgIV molecules comprising an affinity region that is capable of interacting with an epitope of a cross-β structure and/or with an epitope of a protein comprising a cross-β structure.

6. A collection of IgIV molecules according to claim 5, selected by a method according to any one of claims 1-4.

7. A composition comprising at least 5, preferably at least 8, more preferably at least 10 isolated, synthetic and/or recombinant molecules comprising an affinity region that is capable of interacting with an epitope of a cross-β structure and/or with an epitope of a protein comprising a cross-β structure.

8. A composition according to claim 7, comprising a functional part, derivative and/or analogue of at least one IgIV molecule comprising an affinity region capable of interacting with an epitope of a cross-β structure and/or with an epitope of a protein comprising a cross-β structure.

9. A composition according to claim 7 or 8, wherein at least one of said molecules further comprises a cross-β structure binding molecule.

10. A composition according to any one of claims 7-9, wherein at least one of said molecules further comprises an effector molecule.

11. A composition according to claim 10, wherein said effector molecule is a protease or a cross-β structure-binding part thereof.

12. A composition according to claim 10, wherein said effector molecule is an immunopotentiating compound, preferably a cytokine.

13. A composition according to claim 10, wherein said effector molecule is a complement activating factor.

14. A composition according to claim 10, wherein said effector molecule is a clearance signal, preferably at least part of a Fc domain.

15. A composition according to claim 10, wherein said effector molecule is an inflammation suppressive compound, preferably a complement inhibiting factor.

16. A composition according to claim 10, wherein said effector molecule is a cross-β structure binding-potentiating factor.

17. A composition according to claim 10, wherein said effector molecule is an opsonizing compound.

18. A composition according to any one of claims 7-17, wherein said isolated, synthetic and/or recombinant molecule is an opsonizing compound.

19. A method for producing a composition according to any one of claims 7-18, comprising defining the amino acid sequence of an affinity region of at least one IgIV molecule capable of interacting with an epitope of a cross-β structure and/or with an epitope of a protein comprising a cross-β structure, and producing isolated, synthetic and/or recombinant molecules comprising said amino acid sequence.

20. A method for selecting from a collection of IgIV molecules according to claim 5 or 6, or from a composition according to any one of claims 7-18, a molecule comprising an affinity region which is capable, upon interacting with an epitope of a cross-β structure and/or upon interacting with an epitope of a protein comprising a cross-β structure, of inducing opsonization of said cross-β structure and/or protein by a phagocytic cell, said method comprising:
- contacting a collection of IgIV molecules according to claim 5 or 6, or a composition according to any one of claims 7-18, with a cross-β structure and/or with a protein comprising a cross-β structure;
- contacting any complex comprising a cross-β structure and/or a protein comprising a cross-β structure, bound to an IgIV molecule and/or to an isolated, synthetic and/or recombinant molecule, with a phagocytic cell; and
- collecting an IgIV molecule and/or isolated, synthetic and/or recombinant molecule that is capable of inducing or enhancing phagocytosis, by a phagocytic cell, of said cross-β structure and/or protein comprising a cross-β structure.

21. A collection of IgIV molecules according to claim 5 or 6, and/or a composition according to any one of claims 7-18, for use as a medicament.

22. Use of a collection of IgIV molecules according to claim 5 or 6, and/or a composition according to any one of claims 7-18, for the manufacture of a medicament for at least partial prevention and/or treatment of a cross-β structure related and/or associated disease, a blood coagulation disorder and/or a microbial/pathogen/bacterial/parasite/viral infection.

23. Use according to claim 22, wherein said microbial/pathogen/bacterial/parasite /viral infection comprises an HIV-related opportunistic infection.

24. A method for increasing extracellular protein degradation and/or protein clearance in an individual, comprising administering a collection of IgIV molecules according to claim 5 or 6, and/or a composition according to any one of claims 7-18, to said individual.

25. A method for at least in part inhibiting cross-β structure mediated effects in an individual, comprising administering an effective amount of a collection of IgIV molecules according to claim 5 or 6, and/or a composition according to any one of claims 7-18 to an individual.

26. A method for at least partial prevention and/or treatment of a cross-β structure related and/or associated disease, a blood coagulation disorder and/or a microbial/pathogen/bacterial/parasite /viral infection in an individual, comprising administering a collection of IgIV molecules according to claim 5 or 6, and/or a composition according to any one of claims 7-18 to said individual.

27. A method for at least partial prevention and or treatment of an HIV-related opportunistic infection in an individual, comprising administering a collection of IgIV molecules according to claim 5 or 6, and/or a composition according to any one of claims 7-18, to said individual.

28. A composition comprising:
- a collection of IgIV molecules according to claim 5 or 6, and/or
- a composition according to any one of claims 7-18, and
- a suitable carrier, diluent and/or excipient.

29. A composition according to claim 28, further comprising a cross-β structure-binding compound.

30. A composition according to claim 28 or 29, further comprising a cross-β structure-binding-potentiating compound.

31. A composition according to any one of claims 28-30, further comprising a complement activating compound.

32. A composition according to any one of claims 28-30, further comprising an immunopotentiating compound, an inflammation suppressive compound, and/or a complement inhibiting compound.

33. Use of a collection of IgIV molecules according to claim 5 or 6, and/or a composition according to any one of claims 7-18, for inhibiting protein-induced blood-platelet aggregation.

34. Use of a collection of IgIV molecules according to claim 5 or 6, and/or a composition according to any one of claims 7-18, for competing binding of tissue type plasminogen activator (tPA) to a cross-β structure and/or to a molecule comprising a cross-β structure.

35. A method for at least partially removing cross-β structures and/or proteins comprising a cross-β structure from a sample, said method comprising contacting a sample with a collection of IgIV molecules according to claim 5 or 6, and/or a composition according to any one of claims 7-18, and removing from said sample a complex of a cross-β structure, and/or protein comprising a cross-β structure, bound to an IgIV molecule and/or an isolated, synthetic and/or recombinant molecule.

36. A method according to claim 35, wherein said sample is a fluid sample.

37. A method according to claim 36, wherein said fluid comprises a body fluid.

38. A method according to claim 35 or 36, wherein said fluid comprises a pharmaceutical or any of its constituents.

39. A method according to claim 35 or 36, wherein said fluid comprises a food substance.

40. A method according to any one of claims 35-39, wherein said collection of IgIV molecules and/or composition is bound to a solid support.

41. A diagnostic kit comprising:
- at least one affinity region of a collection of IgIV molecules according to claim 5 or 6, and/or at least one affinity region of a composition according to any one of claims 7-18, capable of interacting with a cross-β structure and/or with a protein comprising a cross-β structure, and
- a way of visualization of an interaction of said cross-β structure and/or said protein with said affinity region.

42. A diagnostic kit according to claim 41, wherein said cross-β structure comprises a disease-related cross-β structure.

43. A method for determining whether a protein and/or peptide comprising a cross-β structure is present in an aqueous solution comprising a protein, said method comprising:
- contacting said aqueous solution with a collection of IgIV molecules according to claim 5 or 6, and/or a composition according to any one of claims 7-18, and
- detecting whether bound protein and/or peptide comprising a cross-β structure is present.

44. A method according to claim 43, wherein said aqueous solution comprises a detergent, a food product, a food supplement, a cell culture medium, a commercially available protein solution used for research purposes, blood, a blood product, cerebrospinal fluid, synovial fluid, lymph fluid, a cosmetic product, a cell, a pharmaceutical composition or any of its constituents comprising a protein, or a combination of any of these.

45. A method for removing a cross-β structure and/or a protein comprising a cross-β structure from a pharmaceutical composition or any of its constituents comprising a protein, said method comprising:
- contacting said pharmaceutical composition or any of its constituents comprising a protein with a collection of IgIV molecules according to claim 5 or 6, and/or with a composition according to any one of claims 7-18;
- allowing binding of said protein and/or peptide comprising a cross-β structure to said collection of IgIV molecules and/or composition; and
- separating bound protein and/or peptide comprising a cross-β structure from said pharmaceutical composition or any of its constituents comprising a protein.

46. A method for decreasing and/or preventing undesired side effects of a pharmaceutical composition and/or increasing the specific activity per gram protein, said method comprising removing an unfolded protein, an unfolded peptide, a misfolded protein, a denatured protein, an aggregated protein, an aggregated peptide, a multimerized protein and/or a multimerized peptide, comprising a cross-β structure, from said pharmaceutical composition or any of its constituents, using a method according to claim 45.

47. A pharmaceutical composition or any of its constituents comprising a protein, obtainable by a method according to claim 45 or 46.

48. Use of a collection of IgIV molecules according to claim 5 or 6, and/or a composition according to any one of claims 7-18, for diminishing accumulation of misfolded protein comprising a cross-β structure.

49. Use according to claim 48, wherein said misfolded protein comprising a cross-β structure is involved in a conformational disease.

50. Use according to claim 49, wherein said disease is an amyloidosis type disease, atherosclerosis, diabetes, bleeding, thrombosis, cancer, sepsis and other inflammatory diseases, rheumatoid arthritis, transmissible spongiform encephalopathies, Multiple Sclerosis, auto-immune diseases, disease associated with loss of memory or Parkinson's disease and other neuronal diseases (epilepsy), encephalopathy, and/or rheuma.

51. Use of a collection of IgIV molecules according to claim 5 or 6, and/or a composition according to any one of claims 7-18, for determining the presence of accumulated deposited misfolded protein with a cross-β structure, preferably involved in a conformational disease.

52. A separation device for carrying out a method according to any one of claims 35-40, or 45-46, said device comprising a system for transporting (circulating) fluids, said system being provided with means for connecting to a flowing fluid, preferably to an individual's circulation, for entry of fluid into said system and return of fluid from said system, preferably to an individual's circulation, said system further comprising a solid phase, said solid phase comprising a collection of IgIV molecules according to claim 5 or 6, and/or a composition according to any one of claims 7-19.

53. A separation device according to claim 52, which is a dialysis apparatus.

54. A method for interfering in coagulation of blood comprising providing to blood a collection of IgIV molecules according to claim 5 or 6, and/or a composition according to any one of claims 7-18.

55. A method for determining the amount of cross-β structures in a composition, preferably in a medicament and/or vaccine, comprising:
- contacting said composition with a collection of IgIV molecules according to claim 5 or 6, and/or with a composition according to any one of claims 7-18, and
- relating the amount of bound cross-β structures to the amount of cross-β structures present in said composition.

56. A method for determining a difference in the cross-β structure content of a protein in a reference sample compared to the cross-β structure content of said protein in a test sample, wherein said test sample has been subjected to a treatment that is expected to have an effect on the cross-β structure content of said protein, the method comprising:
- determining in a reference sample the cross-β structure content of a protein using a collection of IgIV molecules according to claim 5 or 6 and/or a composition according to any one of claims 7-18;
- subjecting said protein to a treatment that is expected to have an effect on the cross-β structure content of said protein, thus obtaining a test sample;
- determining in the obtained test sample the cross-β structure content of said protein using a collection of IgIV molecules according to claim 5 or 6, and/or a composition according to any one of claims 7-18; and
- determining whether the cross-β structure content of said protein in said reference sample is significantly different from the cross-β structure content of said protein in said test sample.

57. A method for determination of the identity of a cross-β structure or a protein comprising a cross-β structure in a sample comprising a protein, said method comprising:
- contacting said sample with a collection of IgIV molecules according claim 5 or 6, and/or a composition according to any one of claims 7-18, resulting in bound cross-β structures and/or bound protein(s) comprising a cross-β structure, and
- identifying a bound cross-β structure and/or bound protein(s) comprising a cross-β structure.

58. A method according to claim 57, wherein said sample comprises an aqueous solution, preferably a body fluid.

59. A method according to claim 57 or 58, wherein body fluids originating from healthy individuals and body fluids from individuals suffering from, or suspected to suffer from, a disease related to and/or associated with the presence of a cross-β structure, are used.
